(19)

Europäisches
Patentamt
European
Patent Office
Office européen
des brevets

(11) **EP 4 205 123 B1**

(12) **EUROPEAN PATENT SPECIFICATION**

(45) Date of publication and mention
of the grant of the patent:
**22.01.2025 Bulletin 2025/04**

(21) Application number: **21777894.3**

(22) Date of filing: **26.08.2021**

(51) International Patent Classification (IPC):
**G16B 30/00** *(2019.01)*  **G16B 40/20** *(2019.01)*
**G16B 50/30** *(2019.01)*  **G16B 40/10** *(2019.01)*
**G16B 40/30** *(2019.01)*

(52) Cooperative Patent Classification (CPC):
**G16B 30/00; G16B 40/10; G16B 40/20;
G16B 40/30**

(86) International application number:
**PCT/US2021/047763**

(87) International publication number:
**WO 2022/047038 (03.03.2022 Gazette 2022/09)**

(54) **DETECTING AND FILTERING CLUSTERS BASED ON ARTIFICIAL INTELLIGENCE-PREDICTED BASE CALLS**

ERKENNUNG UND FILTERUNG VON CLUSTERN AUF BASIS VON DURCH KÜNSTLICHE INTELLIGENZ VORHERGESAGTEN BASISANRUFEN

DÉTECTION ET FILTRAGE DE GROUPES SUR LA BASE D'APPELS DE BASE PRÉDITS PAR INTELLIGENCE ARTIFICIELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.08.2020 US 202063072032 P
25.08.2021 US 202117411980**

(43) Date of publication of application:
**05.07.2023 Bulletin 2023/27**

(73) Proprietor: **Illumina, Inc.
San Diego, CA 92122 (US)**

(72) Inventors:
• **KASHEFHAGHIGHI, Dorna
San Francisco, California 94158 (US)**
• **PARNABY, Gavin Derek
San Diego, California 92122 (US)**

(74) Representative: **Robinson, David Edward
Ashdown
Marks & Clerk LLP
1 New York Street
Manchester M1 4HD (GB)**

(56) References cited:
**WO-A1-2019/140146**    **WO-A1-2020/014280**
**US-A1- 2015 169 824**    **US-A1- 2017 161 900**
**US-A1- 2018 121 601**    **US-A1- 2018 195 953**
**US-A1- 2018 274 023**

• **NIEMI JARAD ET AL: "Probabilistic methods for
quality improvement in high-throughput
sequencing", 1 January 2016 (2016-01-01),
XP055870547, [retrieved on 20211208]**
• **WEI-CHUN KAO: "Algorithms for Next-
Generation High-Throughput Sequencing
Technologies", 1 January 2011 (2011-01-01),
XP055360975, ISBN: 978-1-267-22655-6,
Retrieved from the Internet
<URL:http://digitalassets.lib.berkeley.
edu/etd/ucb/text/Kao_berkeley_0028E_11836.
pdf>**

- ANONYMOUS: "MiSeq: Imaging and Base Calling", 1 January 2013 (2013-01-01), XP055669460, Retrieved from the Internet <URL:https://support.illumina. com/content/dam/illumina-support/courses/Mi Seq_Imaging_and_Base_Calling/story_conten t/external_files/MiSeq%20Imaging%20and% 20Base%20Calling%20Script.pdf> [retrieved on 20200218]
- MAUNG WAI PHYO ET AL: "DNA Sequence Analyses and Error Correction: A Major Qualifying Project TABLE OF CONTENT", 30 April 2014 (2014-04-30), XP055870191, [retrieved on 20211207]
- KIRCHER MARTIN ET AL: "Improved base calling for the Illumina Genome Analyzer using machine learning strategies", GENOME BIOLOGY, BIOMED CENTRAL LTD, vol. 10, no. 8, 14 August 2009 (2009-08-14), pages R83, XP021061445, ISSN: 1465-6906, DOI: 10.1186/GB-2009-10-8-R83
- ANONYMUS: "NovaSeq 6000 - Sequencing System Guide", 1 February 2019 (2019-02-01), XP055656862, Retrieved from the Internet <URL:https://support.illumina. com/content/dam/illumina-support/documents/ documentation/system_documentation/nova seq/novaseq-6000-system- guide-1000000019358-11.pdf> [retrieved on 20200110]
- ANONYMOUS: "NextSeq 500 system guide", ILLUMINA, 1 October 2015 (2015-10-01), XP055517630
- WEI-CHUN KAO ET AL: "naiveBayesCall: An Efficient Model-Based Base-Calling Algorithm for High-Throughput Sequencing", 25 April 2010, RESEARCH IN COMPUTATIONAL MOLECULAR BIOLOGY, SPRINGER BERLIN HEIDELBERG, BERLIN, HEIDELBERG, PAGE(S) 233 - 247, ISBN: 978-3-642-12682-6, XP019141683
- ASHLEY CACHO ET AL: "A Comparison of Base-calling Algorithms for Illumina Sequencing Technology", vol. 17, no. 5, 1 September 2016 (2016-09-01), pages 786 - 795, XP009504597, ISSN: 1467-5463, Retrieved from the Internet <URL:https://academic.oup. com/bib/article-abstract/17/5/786/2262186> [retrieved on 20151005], DOI: 10.1093/BIB/ BBV088

**EP 4 205 123 B1**

**Description**

Related disclosures:

U.S. Provisional Patent Application No. 62/821,602, entitled "Training Data Generation for Artificial Intelligence-Based Sequencing," filed 21 March 2019 (Attorney Docket No. ILLM 1008-1/IP-1693-PRV);
U.S. Provisional Patent Application No. 62/821,618, entitled "Artificial Intelligence-Based Generation of Sequencing Metadata," filed 21 March 2019 (Attorney Docket No. ILLM 1008-3/IP-1741-PRV);
U.S. Provisional Patent Application No. 62/821,681, entitled "Artificial Intelligence-Based Base Calling," filed 21 March 2019 (Attorney Docket No. ILLM 1008-4/IP-1744-PRV);
U.S. Provisional Patent Application No. 62/821,724, entitled "Artificial Intelligence-Based Quality Scoring," filed 21 March 2019 (Attorney Docket No. ILLM 1008-7/IP-1747-PRV);
U.S. Provisional Patent Application No. 62/821,766, entitled "Artificial Intelligence-Based Sequencing," filed 21 March 2019 (Attorney Docket No. ILLM 1008-9/IP-1752-PRV);
NL Application No. 2023310, entitled "Training Data Generation for Artificial Intelligence-Based Sequencing," filed 14 June 2019 (Attorney Docket No. ILLM 1008-11/IP-1 693-NL);
NL Application No. 2023311, entitled "Artificial Intelligence-Based Generation of Sequencing Metadata," filed 14 June 2019 (Attorney Docket No. ILLM 1008-12/IP-1741-NL);
NL Application No. 2023312, entitled "Artificial Intelligence-Based Base Calling," filed 14 June 2019 (Attorney Docket No. ILLM 1008-13/IP-1744-NL);
NL Application No. 2023314, entitled "Artificial Intelligence-Based Quality Scoring," filed 14 June 2019 (Attorney Docket No. ILLM 1008-14/IP-1747-NL);
NL Application No. 2023316, entitled "Artificial Intelligence-Based Sequencing," filed 14 June 2019 (Attorney Docket No. ILLM 1008-15/IP-1752-NL);
U.S. Provisional Patent Application No. 62/849,091, entitled," Systems and Devices for Characterization and Performance Analysis of Pixel-Based Sequencing," filed May 16, 2019 (Attorney Docket No. ILLM 1011-1/IP-1750-PRV);
U.S. Provisional Patent Application No. 62/849,132, entitled, "Base Calling Using Convolutions," filed May 16, 2019 (Attorney Docket No. ILLM 1011-2/IP-1750-PR2);
U.S. Provisional Patent Application No. 62/849,133, entitled, "Base Calling Using Compact Convolutions," filed May 16, 2019 (Attorney Docket No. ILLM 1011-3/IP-1750-PR3);
U.S. Provisional Patent Application No. 62/979,384, entitled, "Artificial Intelligence-Based Base Calling of Index Sequences," filed February 20, 2020 (Attorney Docket No. ILLM 1015-1/IP-1857-PRV);
U.S. Provisional Patent Application No. 62/979,414, entitled, "Artificial Intelligence-Based Many-To-Many Base Calling," filed February 20, 2020 (Attorney Docket No. ILLM 1016-1/IP-1858-PRV);
U.S. Provisional Patent Application No. 62/979,385, entitled, "Knowledge Distillation-Based Compression of Artificial Intelligence-Based Base Caller," filed February 20, 2020 (Attorney Docket No. ILLM 1017-1/IP-1859-PRV);
U.S. Provisional Patent Application No. 62/979,412, entitled, "Multi-Cycle Cluster Based Real Time Analysis System," filed February 20, 2020 (Attorney Docket No. ILLM 1020-1/IP-1866-PRV);
U.S. Provisional Patent Application No. 62/979,411, entitled, "Data Compression for Artificial Intelligence-Based Base Calling," filed February 20, 2020 (Attorney Docket No. ILLM 1029-1/IP-1964-PRV); and
U.S. Provisional Patent Application No. 62/979,399, entitled, "Squeezing Layer for Artificial Intelligence-Based Base Calling," filed February 20, 2020 (Attorney Docket No. ILLM 1030-1/IP-1982-PRV).

## FIELD OF THE TECHNOLOGY DISCLOSED

The technology disclosed relates to artificial intelligence type computers and digital data processing systems and corresponding data processing methods and products for emulation of intelligence (i.e., knowledge based systems, reasoning systems, and knowledge acquisition systems); and including systems for reasoning with uncertainty (e.g., fuzzy logic systems), adaptive systems, machine learning systems, and artificial neural networks. In particular, the technology disclosed relates to using deep neural networks such as deep convolutional neural networks for analyzing data.

## BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

Base calling assigns bases and associated quality values for each position of the read. The quality of the sequenced bases is assessed by Illumina sequencers with a procedure called chastity filter. Chastity can be determined as the highest intensity value divided by the sum of the highest intensity value and the second highest intensity value. Quality evaluation can include identifying reads where the second worst chastity in the first subset of base calls is below a threshold and marking those reads as poor quality data. The first subset of base calls can be any suitable number of base calls. For example, the subset can be the first 1, 2, 3, 4, 5, 6, 7, 8, 9, 10, 11, 12, 13, 14, 15, 16, 17, 18, 19, 20, 21, 22, 23, 24, 25 or greater than the first 25 base calls. This can be termed read filtering, such that clusters that meet this cutoff are referred to as having "passed filter".

$$Chastity = \frac{Highest\ intensity}{Highest\ intensity + Next\ highest\ intensity}$$

In some implementations, the purity of the signal from each cluster is examined over the first twenty-five cycles and calculated as the chastity value. At most one cycle may fall below the chastity threshold (e.g., 0.6), otherwise, the read will not pass the chastity filter.

Illumina calculates a Phred score that is used to store an assessment for the error probability of a base call. The Phred score is computed based on intensity profiles (shifted purity: how much of signal is accounted for by the brightest channel?) and signal to noise ratios (signal overlap with the background: is the signal from the colony well delineated from the surrounding region of the flow cell?). Illumina attempts to quantify the chastity of the strongest base signal, whether a signal for a given base call is much stronger than that of nearby bases, whether a spot representing a colony gets suspiciously dim during the course of sequencing (intensity decay), and whether the signal in the preceding and following cycles appears clean or not.

US 2018/274023 A1 discloses methods and systems for analysis of image data generated from various reference points useful for real time analysis of image and sequence data generated during DNA sequencing methodologies.

The article by Wei-Chun KAO et al.: "naiveBayesCall: An Efficient Model-Based Base- Calling Algorithm for High-Throughput Sequencing", 25 April 2010 (2010-04-25), Research In Computational Molecular Biology, Springer, Berlin, Heidelberg, pages 233 - 247, XP019141683, ISBN: 978-3-642-12682-6, discloses efficient algorithms for base calling to reduce the error rate, particularly in the later cycles of the sequencing run and provides useful base-specific quality scores with high discrimination ability.

An opportunity arises to detect and filter unreliable clusters based on artificial intelligence-predicted base calls. Improved base calling accuracy and quality may result.

## BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which.

Figure 1 is a block diagram that shows various aspects of the technology disclosed.
Figure 2A shows an example softmax function.
Figure 2B depicts example per-cluster, per-cycle probability quadruples produced by the technology disclosed.
Figure 3 shows an example of identifying unreliable clusters using filter values.
Figure 4 is a flowchart illustrating one implementation of a method of identifying unreliable clusters to improve accuracy and efficiency of base calling d.
Figures 5A and 5B depict one implementation of a sequencing system. The sequencing system comprises a configurable processor.
Figure 5C is a simplified block diagram of a system for analysis of sensor data from the sequencing system, such as base call sensor outputs.
Figure 6 shows one implementation of the disclosed data flow logic that enables a host processor to filter unreliable clusters based on base calls predicted by a neural network running on a configurable processor, and further enables the configurable processor to use data identifying the unreliable clusters to generate reliable remainder intermediate representations.
Figure 7 shows another implementation of the disclosed data flow logic that enables the host processor to filter unreliable clusters based on base calls predicted by the neural network running on the configurable processor, and further enables the host processor to use data identifying the unreliable clusters to base call only reliable clusters.

Figure 8 shows yet another implementation of the disclosed data flow logic that enables the host processor to filter unreliable clusters based on base calls predicted by the neural network running on the configurable processor, and further uses data identifying the unreliable clusters to generate reliable remainder per-cluster data.

Figures 9, 10, 11, 12, and 13 show results of comparative analysis of detection of empty and non-empty wells by the technology disclosed referred to herein as "DeepRTA" versus Illumina's traditional base caller called Real-Time Analysis (RTA) software.

Figure 14 is a computer system that can be used to implement the technology disclosed.

## DETAILED DESCRIPTION

The following discussion is presented to enable any person skilled in the art to make and use the technology disclosed and is provided in the context of a particular application and its requirements. Various modifications to the disclosed implementations will be readily apparent to those skilled in the art. The invention is defined by the appended claims.

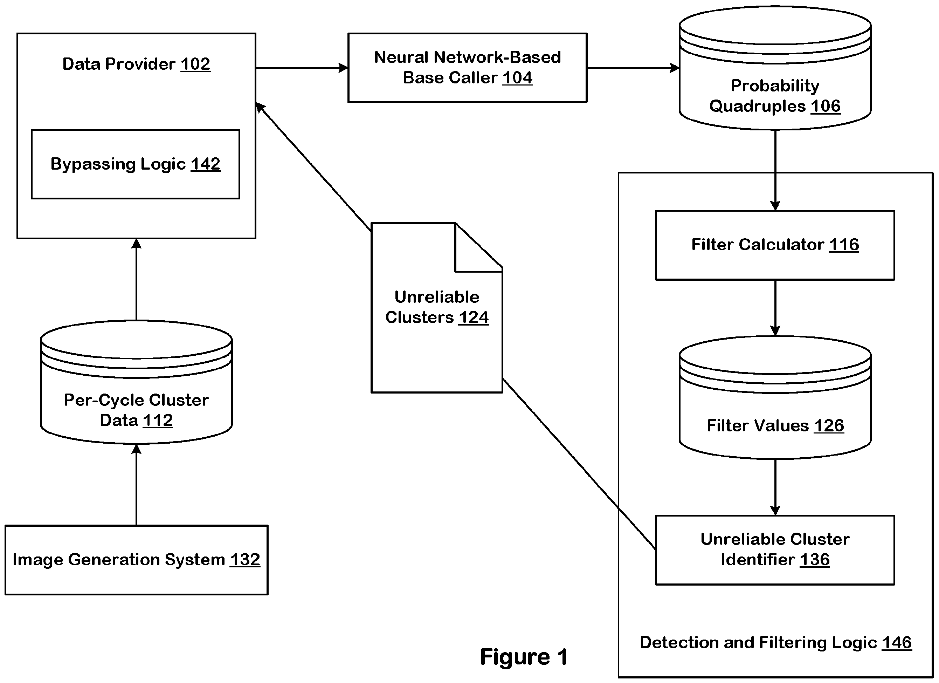

This disclosure provides methods and systems of artificial intelligence-based image analysis that are particularly useful for detecting and filtering unreliable clusters. Figure 1 illustrates an example data analysis and filtering system and certain of its components. The system includes an image generation system 132, per-cycle cluster data 112, a data provider 102, a neural network-based base caller 104, probability quadruples 106, detection and filtering logic 146, and data identifying unreliable clusters 124. The system can be formed by one or more programmed computers, with programming being stored on one or more machine readable media with code executed to carry out one or more steps of methods described herein. In the illustrated implementation, for example, the system includes the image generation system 132 configured to output the per-cycle cluster data 112 as digital image data, for example, image data that is representative of individual picture elements or pixels that, together, form an image of an array or other object.

## Neural Network-Based Base Calling

Base calling is the process of determining the nucleotide composition of a sequence. Base calling involves analyzing image data, i.e., sequencing images produced during the sequencing reaction carried out by a sequencing instrument such as Illumina's iSeq, HiSeqX, HiSeq 3000, HiSeq 4000, HiSeq 2500, NovaSeq 6000, NextSeq 550, NextSeq 1000, NextSeq 2000, NextSeqDx, MiSeq, and MiSeqDx. The following discussion outlines how the sequencing images are generated and what they depict, in accordance with one implementation.

Base calling decodes the raw signal of the sequencing instrument, i.e., intensity data extracted from the sequencing images, into nucleotide sequences. In one implementation, the Illumina platforms employ cyclic reversible termination (CRT) chemistry for base calling. The process relies on growing nascent strands complementary to template strands with fluorescently-labeled nucleotides, while tracking the emitted signal of each newly added nucleotide. The fluorescently-labeled nucleotides have a 3' removable block that anchors a fluorophore signal of the nucleotide type.

Sequencing occurs in repetitive cycles, each comprising three steps: (a) extension of a nascent strand by adding the fluorescently-labeled nucleotide; (b) excitation of the fluorophore using one or more lasers of an optical system of the sequencing instrument and imaging through different filters of the optical system, yielding the sequencing images; and (c) cleavage of the fluorophore and removal of the 3' block in preparation for the next sequencing cycle. Incorporation and imaging cycles are repeated up to a designated number of sequencing cycles, defining the read length. Using this approach, each cycle interrogates a new position along the template strands.

The tremendous power of the Illumina sequencers stems from their ability to simultaneously execute and sense millions or even billions of clusters (e.g., clusters) undergoing CRT reactions. A cluster comprises approximately one thousand identical copies of a template strand, though clusters vary in size and shape. The clusters are grown from the template strand, prior to the sequencing run, by bridge amplification or exclusion amplification of the input library. The purpose of the amplification and cluster growth is to increase the intensity of the emitted signal since the imaging device cannot reliably sense fluorophore signal of a single strand. However, the physical distance of the strands within a cluster is small, so the imaging device perceives the cluster of strands as a single spot.

Sequencing occurs in a flow cell - a small glass slide that holds the input strands. The flow cell is connected to the optical system, which comprises microscopic imaging, excitation lasers, and fluorescence filters. The flow cell comprises multiple chambers called lanes. The lanes are physically separated from each other and may contain different tagged sequencing libraries, distinguishable without sample cross contamination. The imaging device of the sequencing instrument (e.g., a solid-state imager such as a charge-coupled device (CCD) or a complementary metal-oxide-semiconductor (CMOS) sensor) takes snapshots at multiple locations along the lanes in a series of non-overlapping regions called tiles. For example, there are hundred tiles per lane in Illumina's Genome Analyzer II and sixty-eight tiles per lane in Illumina's HiSeq 2000. A tile holds hundreds of thousands to millions of clusters.

The output of the sequencing is the sequencing images, each depicting intensity emissions of the clusters and their surrounding background. The sequencing images depict intensity emissions generated as a result of nucleotide incorporation in the sequences during the sequencing. The intensity emissions are from associated clusters and their surrounding background.

The following discussion is organized as follows. First, the input to the neural network-based base caller 104 is described, in accordance with one implementation. Then, examples of the structure and form of the neural network-based base caller 104 are provided. Finally, the output of the neural network-based base caller 104 is described, in accordance with one implementation.

Additional details about the neural network-based base caller 104 can be found in US Provisional Patent Application No. 62/821,766, titled "ARTIFICIAL INTELLIGENCE-BASED SEQUENCING," (Attorney Docket No. ILLM 1008-9/IP-1752-PRV), filed on March 21, 2019.

In one implementation, image patches are extracted from the sequencing images. Data provider 102 provides the extracted image patches to the neural network-based base caller 104 as "input image data" for base calling. The image patches have dimensions $w \times h$, where w (width) and $h$ (height) are any numbers ranging from 1 and 10,000 (e.g., 3 x 3, 5 x 5, 7 x 7, 10 x 10, 15 x 15, 25 x 25). In some implementations, $w$ and h are the same. In other implementations, $w$ and $h$ are different.

Sequencing produces $m$ image(s) per sequencing cycle for corresponding $m$ image channels. In one implementation, each image channel corresponds to one of a plurality of filter wavelength bands. In another implementation, each image channel corresponds to one of a plurality of imaging events at a sequencing cycle. In yet another implementation, each image channel corresponds to a combination of illumination with a specific laser and imaging through a specific optical filter.

An image patch is extracted from each of the $m$ image(s) to prepare the input image data for a particular sequencing cycle. In different implementations such as 4-, 2-, and 1-channel chemistries, $m$ is 4 or 2. In other implementations, $m$ is 1, 3, or greater than 4. The input image data is in the optical, pixel domain in some implementations, and in the upsampled, subpixel domain in other implementations.

Consider, for example, that sequencing uses two different image channels: a red channel and a green channel. Then, at each sequencing cycle, sequencing produces a red image and a green image. This way, for a series of $k$ sequencing cycle, a sequence with $k$ pairs of red and green images is produced as output.

The input image data comprises a sequence of per-cycle image patches generated for a series of $k$ sequencing cycles of a sequencing run. The per-cycle image patches contain intensity data for associated clusters and their surrounding background in one or more image channels (e.g., a red channel and a green channel). In one implementation, when a single target cluster (e.g., cluster) is to be base called, the per-cycle image patches are centered at a center pixel that contains intensity data for a target associated cluster and non-center pixels in the per-cycle image patches contain intensity data for associated clusters adjacent to the target associated cluster. The per-cycle image patches for a plurality of sequencing cycles are stored as per-cycle cluster data 112.

The input image data comprises data for multiple sequencing cycles (e.g., a current sequencing cycle, one or more preceding sequencing cycles, and one or more successive sequencing cycles). In one implementation, the input image data comprises data for three sequencing cycles, such that data for a current (time t) sequencing cycle to be base called is accompanied with (i) data for a left flanking/context/previous/preceding/prior (time t-1) sequencing cycle and (ii) data for a right flanking/context/next/successive/subsequent (time $t$+1) sequencing cycle. In another implementation, the input image data comprises data for five sequencing cycles, such that data for a current (time $t$) sequencing cycle to be base called is accompanied with (i) data for a first left flanking/context/previous/preceding/prior (time $t$-1) sequencing cycle, (ii) data for a second left flanking/context/previous/preceding/prior (time $t$-2) sequencing cycle, (iii) data for a first right flanking/context/next/successive/subsequent (time $t$+1), and (iv) data for a second right flanking/context/next/successive/subsequent (time $t$+2) sequencing cycle. In yet another implementation, the input image data comprises data for seven sequencing cycles, such that data for a current (time $t$) sequencing cycle to be base called is accompanied with (i) data for a first left flanking/context/previous/preceding/prior (time $t$-1) sequencing cycle, (ii) data for a second left flanking/context/previous/preceding/prior (time t-2) sequencing cycle, (iii) data for a third left flanking/context/previous/preceding/prior (time $t$-3) sequencing cycle, (iv) data for a first right flanking/context/next/successive/subsequent (time $t$+1), (v) data for a second right flanking/context/next/successive/subsequent (time $t$+2) sequencing cycle, and (vi) data for a third right flanking/context/next/successive/subsequent (time $t$+3) sequencing cycle. In other implementations, the input image data comprises data for a single sequencing cycle. In yet other implementations, the input image data comprises data for 58, 75, 92, 130, 168, 175, 209, 225, 230, 275, 318, 325, 330, 525, or 625 sequencing cycles.

In one implementation, the sequencing images from the current (time t) sequencing cycle are accompanied with the sequencing images from the first and second preceding (time $t$-1, time $t$-2) sequencing cycles and the sequencing images from the first and second succeeding (time $t$+1, time $t$+2) sequencing cycles. The neural network-based base caller 104 processes the sequencing images through its convolution layers and produces an alternative representation, according to one implementation. The alternative representation is then used by an output layer (e.g., a softmax layer) for generating a base call for either just the current (time $t$) sequencing cycle or each of the sequencing cycles, i.e., the current (time $t$) sequencing cycle, the first and second preceding (time $t$-1, time $t$-2) sequencing cycles, and the first and second succeeding (time $t$+1, time $t$+2) sequencing cycles. The resulting base calls form the sequencing reads.

In another implementation, the sequencing images from the current (time $t$) sequencing cycle are accompanied with the sequencing images from the preceding (time $t$-1) sequencing cycle and the sequencing images from the succeeding (time $t$+1) sequencing cycle. The neural network-based base caller 104 processes the sequencing images through its convolution layers and produces an alternative representation, according to one implementation. The alternative representation is then used by an output layer (e.g., a softmax layer) for generating a base call for either just the current (time $t$) sequencing cycle or each of the sequencing cycles, i.e., the current (time $t$) sequencing cycle, the preceding (time $t$-1) sequencing cycle, and the succeeding (time $t$+1) sequencing cycle. The resulting base calls form the sequencing reads.

In one implementation, the neural network-based base caller 104 outputs a base call for a single target cluster for a particular sequencing cycle. In another implementation, it outputs a base call for each target cluster in a plurality of target clusters for the particular sequencing cycle. In yet another implementation, it outputs a base call for each target cluster in a plurality of target clusters for each sequencing cycle in a plurality of sequencing cycles, thereby producing a base call sequence for each target cluster.

In one implementation, the neural network-based base caller 104 is a multilayer perceptron (MLP). In another implementation, the neural network-based base caller 104 is a feedforward neural network. In yet another implementation, the neural network-based base caller 104 is a fully-connected neural network. In a further implementation, the neural network-based base caller 104 is a fully convolutional neural network. In yet further implementation, the neural network-based base caller 104 is a semantic segmentation neural network. In yet another further implementation, the neural network-based base caller 104 is a generative adversarial network (GAN).

In one implementation, the neural network-based base caller 104 is a convolutional neural network (CNN) with a plurality of convolution layers. In another implementation, it is a recurrent neural network (RNN) such as a long short-term memory network (LSTM), bidirectional LSTM (Bi-LSTM), or a gated recurrent unit (GRU). In yet another implementation, it includes both a CNN and a RNN.

In yet other implementations, the neural network-based base caller 104 can use 1D convolutions, 2D convolutions, 3D convolutions, 4D convolutions, 5D convolutions, dilated or atrous convolutions, transpose convolutions, depthwise separable convolutions, pointwise convolutions, $1 \times 1$ convolutions, group convolutions, flattened convolutions, spatial and cross-channel convolutions, shuffled grouped convolutions, spatial separable convolutions, and deconvolutions. It can use one or more loss functions such as logistic regression/log loss, multi-class cross-entropy/-softmax loss, binary cross-entropy loss, mean-squared error loss, L1 loss, L2 loss, smooth L1 loss, and Huber loss. It can use any parallelism, efficiency, and compression schemes such TFRecords, compressed encoding (e.g., PNG), sharding, parallel calls for map transformation, batching, prefetching, model parallelism, data parallelism, and synchronous/asynchronous stochastic gradient descent (SGD). It can include upsampling layers, downsampling layers, recurrent connections, gates and gated memory units (like an LSTM or GRU), residual blocks, residual connections, highway connections, skip connections, peephole connections, activation functions (e.g., non-linear transformation functions like rectifying linear unit (ReLU), leaky ReLU, exponential liner unit (ELU), sigmoid and hyperbolic tangent (tanh)), batch normalization layers, regularization layers, dropout, pooling layers (e.g., max or average pooling), global average pooling layers, and attention mechanisms.

The neural network-based base caller 104 is trained using backpropagation-based gradient update techniques. Example gradient descent techniques that can be used for training the neural network-based base caller 104 include stochastic gradient descent, batch gradient descent, and mini-batch gradient descent. Some examples of gradient descent optimization algorithms that can be used to train the neural network-based base caller 104 are Momentum, Nesterov accelerated gradient, Adagrad, Adadelta, RMSprop, Adam, AdaMax, Nadam, and AMSGrad.

The neural network-based base caller 104 uses a specialized architecture to segregate processing of data for different sequencing cycles. The motivation for using the specialized architecture is described first. As discussed above, the neural network-based base caller 104 processes intensity contextualized patches for a current sequencing cycle, one or more preceding sequencing cycles, and one or more successive sequencing cycles. Data for additional sequencing cycles provides sequence-specific context. The neural network-based base caller 104 learns the sequence-specific context during training and base call them. Furthermore, data for pre and post sequencing cycles provides second order contribution of pre-phasing and phasing signals to the current sequencing cycle.

However, images captured at different sequencing cycles and in different image channels are misaligned and have residual registration error with respect to each other. To account for this misalignment, the specialized architecture comprises spatial convolution layers that do not mix information between sequencing cycles and only mix information within a sequencing cycle.

Spatial convolution layers use so-called "segregated convolutions" that operationalize the segregation by independently processing data for each of a plurality of sequencing cycles through a "dedicated, non-shared" sequence of convolutions. The segregated convolutions convolve over data and resulting feature maps of only a given sequencing cycle, i.e., intra-cycle, without convolving over data and resulting feature maps of any other sequencing cycle.

Consider, for example, that the input data comprises (i) current intensity contextualized patch for a current (time $t$) sequencing cycle to be base called, (ii) previous intensity contextualized patch for a previous (time $t$-1) sequencing cycle, and (iii) next intensity contextualized patch for a next (time $t$+1) sequencing cycle. The specialized architecture then initiates three separate convolution pipelines, namely, a current convolution pipeline, a previous convolution pipeline, and a next convolution pipeline. The current data processing pipeline receives as input the current intensity contextualized patch for the current (time t) sequencing cycle and independently processes it through a plurality of spatial convolution layers 784 to produce a so-called "current spatially convolved representation" as the output of a final spatial convolution layer. The previous convolution pipeline receives as input the previous intensity contextualized patch for the previous (time $t$-1) sequencing cycle and independently processes it through the plurality of spatial convolution layers to produce a so-called "previous spatially convolved representation" as the output of the final spatial convolution layer. The next convolution pipeline receives as input the next intensity contextualized patch for the next (time $t$+1) sequencing cycle and independently processes it through the plurality of spatial convolution layers to produce a so-called "next spatially convolved representation" as the output of the final spatial convolution layer.

In some implementations, the current, previous, and next convolution pipelines are executed in parallel. In some implementations, the spatial convolution layers are part of a spatial convolutional network (or subnetwork) within the specialized architecture.

The neural network-based base caller 104 further comprises temporal convolution layers that mix information between sequencing cycles, i.e., inter-cycles. The temporal convolution layers receive their inputs from the spatial convolutional network and operate on the spatially convolved representations produced by the final spatial convolution layer for the respective data processing pipelines.

The inter-cycle operability freedom of the temporal convolution layers emanates from the fact that the misalignment property, which exists in the image data fed as input to the spatial convolutional network, is purged out from the spatially convolved representations by the stack, or cascade, of segregated convolutions performed by the sequence of spatial convolution layers.

Temporal convolution layers use so-called "combinatory convolutions" that groupwise convolve over input channels in successive inputs on a sliding window basis. In one implementation, the successive inputs are successive outputs produced by a previous spatial convolution layer or a previous temporal convolution layer.

In some implementations, the temporal convolution layers are part of a temporal convolutional network (or subnetwork) within the specialized architecture. The temporal convolutional network receives its inputs from the spatial convolutional network. In one implementation, a first temporal convolution layer of the temporal convolutional network groupwise combines the spatially convolved representations between the sequencing cycles. In another implementation, subsequent temporal convolution layers of the temporal convolutional network combine successive outputs of previous temporal convolution layers. The output of the final temporal convolution layer is fed to an output layer that produces an output. The output is used to base call one or more clusters at one or more sequencing cycles.

In one implementation, bypassing base calling the unreliable clusters refers to processing the unreliable clusters only through the spatial convolution layers of the neural network-based base caller 104, and not processing the unreliable clusters through the temporal convolution layers of the neural network-based base caller 104.

In the context of this application, unreliable clusters are also identified by pixels that do not depict any clusters, and such pixels are discarded from processing by the temporal convolution layers. In some implementations, this occurs when the wells, into which the biological samples are deposited, are empty.


## Detecting and Filtering Unreliable Clusters

The technology disclosed detects and filters unreliable clusters. The following discussion explains unreliable clusters.

Unreliable clusters are low-quality clusters that emit an amount of desired signal which is insignificant compared to background signal. The signal to noise ratio for unreliable clusters is substantially low, for example, less than 1. In some implementations, unreliable clusters may not produce any amount of a desired signal. In other implementations, unreliable clusters may produce a very low amount of signal relative to background. In one implementation, the signal is an optical signal and is intended to include, for example, fluorescent, luminescent, scatter, or absorption signals. Signal level refers to an amount or quantity of detected energy or coded information that has a desired or predefined characteristic. For example, an optical signal can be quantified by one or more of intensity, wavelength, energy, frequency, power luminance or the like. Other signals can be quantified according to characteristics such as voltage, current, electric field strength, magnetic field strength, frequency, power, temperature, etc. Absence of signal in unreliable clusters is understood to be a signal level of zero or a signal level that is not meaningfully distinguished from noise.

There are many potential reasons for poor quality signals of unreliable clusters. If there has been a polymerase chain reaction (PCR) error in colony amplification such that a sizable proportion of the ~1000 molecules in an unreliable cluster contains a different base at a certain position, then one may observe a signal for two bases-this is interpreted as a sign of poor quality and referred to as phase error. Phase error occurs when individual molecules in an unreliable cluster do not incorporate a nucleotide in some cycle (e.g., because of incomplete remove of the 3' terminators, termed phasing) and then lag behind the other molecules, or when an individual molecule incorporates more than one nucleotide in a single cycle (e.g., because of incorporation of nucleotides without effective 3'-blocking, termed prephasing). This results in the loss of synchrony in the readout of the sequence copies. The proportion of sequences in unreliable clusters that are affected by phasing and pre-phasing increases with cycle number, which is a major reason why the quality of reads tends to decline at high cycle numbers.

Unreliable clusters also result from fading. Fading is an exponential decay in signal intensity of unreliable clusters as a function of cycle number. As the sequencing run progress, the strands in unreliable clusters are washed excessively, exposed to laser emissions that create reactive species, and subject to harsh environmental conditions. All of these lead to a gradual loss of fragments in unreliable clusters, decreasing their signal intensity.

Unreliable clusters also result from underdeveloped colonies, i.e., small cluster sizes of unreliable clusters that produce empty or partially filled wells on a patterned flow cell. That is, in some implementations, the unreliable clusters are indicative of empty, polyclonal, and dim wells on the patterned flow cell. Unreliable clusters also result from overlapping colonies caused by unexclusive amplification. Unreliable clusters also result from under-illumination or uneven-illumination, for example, due to being located on the edges of a flow cell. Unreliable clusters also result from impurities on the flow cell that obfuscate emitted signal. Unreliable clusters also include polyclonal clusters when multiple clusters are deposited in the same well.

The discussion now turns to how unreliable clusters are detected and filtered by the detection and filtering logic 146 to improve accuracy and efficiency of base calling. The data provider 102 provides the per-cycle cluster data 112 to the neural network-based base caller 104. The per-cycle cluster data 112 is for a plurality of clusters and for a first subset of sequencing cycles of a sequencing run. Consider, for example, that the sequencing run has 150 sequencing cycles. The first subset of sequencing cycles can then include any subset of the 150 sequencing cycles, for example, the first 5, 10, 15, 25, 35, 40, 50, or 100 sequencing cycles of the 150-cycle sequencing run. Also, each sequencing cycle produces sequencing images that depict intensity emissions of clusters in the plurality of clusters. This way, the per-cycle cluster data 112 for the plurality of clusters and for the first subset of sequencing cycles of the sequencing run includes sequencing images only for the first 5, 10, 15, 25, 35, 40, 50, or 100 sequencing cycles of the 150-cycle sequencing run and does not include sequencing images for the remainder sequencing cycles of the 150-cycle sequencing run.

The neural network-based base caller 104 base calls each cluster in the plurality of clusters at each sequencing cycle in the first subset of sequencing cycles. To do so, the neural network-based base caller 104 processes the per-cycle cluster data 112 and generates intermediate representations of the per-cycle cluster data 112. Then, the neural network-based base caller 104 processes the intermediate representations though an output layer and produces a per-cluster, per-cycle probability quadruple for each cluster and for each sequencing cycle. Examples of the output layer include a softmax function, a log-softmax function, an ensemble output average function, a multi-layer perceptron uncertainty function, a Bayes Gaussian distribution function, and a cluster intensity function. The per-cluster, per-cycle probability quadruples are stored as the probability quadruples 106.

The following discussion focuses on the per-cluster, per-cycle probability quadruples using the softmax function as an example. We first explain the softmax function and then the per-cluster, per-cycle probability quadruples.

Softmax function is a preferred function for multi-class classification. The softmax function calculates the probabilities of each target class over all possible target classes. The output range of the softmax function is between zero and one and the sum of all the probabilities is equal to one. The softmax function computes the exponential of the given input value and the sum of exponential values of all the input values. The ratio of the exponential of the input value and the sum of exponential values is the output of the softmax function, referred to herein as "exponential normalization."

Formally, training a so-called softmax classifier is regression to a class probability, rather than a true classifier as it does not return the class but rather a confidence prediction of each class's probability. The softmax function takes a class of values and converts them to probabilities that sum to one. The softmax function squashes a $n$-dimensional vector of arbitrary real values to $n$-dimensional vector of real values within the range zero to one. Thus, using the softmax function ensures that the output is a valid, exponentially normalized probability mass function (nonnegative and summing to one).

Intuitively, the softmax function is a "soft" version of the maximum function. The term "soft" derives from the fact that the softmax function is continuous and differentiable. Instead of selecting one maximal element, it breaks the vector into parts of a whole with the maximal input element getting a proportionally larger value, and the other getting a less proportion of the value. The property of outputting a probability distribution makes the softmax function suitable for probabilistic interpretation in classification tasks.

Let us consider $z$ as a vector of inputs to the softmax layer. The softmax layer units are the number of nodes in the softmax layer and therefore, the length of the $z$ vector is the number of units in the softmax layer (if we have ten output units, then there are ten $z$ elements).

For an $n$-dimensional vector $Z = [z_1, z_2, ... z_n]$, the softmax function uses exponential normalization (exp) to produce another *n*- dimensional vector *p(Z)* with normalized values in the range [0, 1] and that add to unity:

$$Z = \begin{bmatrix} z_1 \\ z_2 \\ \vdots \\ z_n \end{bmatrix} and, \ p(Z) \ \rightarrow \ \begin{bmatrix} p_1 \\ p_2 \\ \vdots \\ p_n \end{bmatrix}$$

$$p_j = \frac{\exp^{z_j}}{\sum\limits_{k=1}^{n} \exp^{z_k}} \ \forall \ j \ \in \ 1, 2, ..., n$$

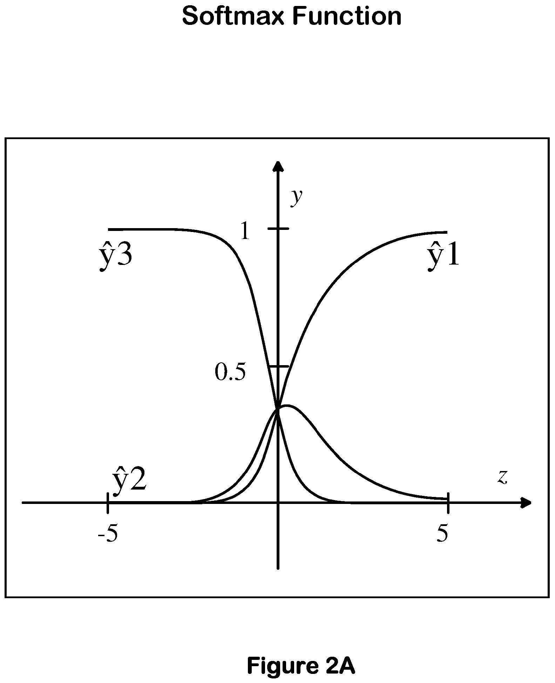

Figure 2A shows an example softmax function. Softmax function is applied to three classes as $z \mapsto$ softmax ([z; - ; -2z]) . Note that the three outputs always sum to one. They thus define a discrete probability mass function.

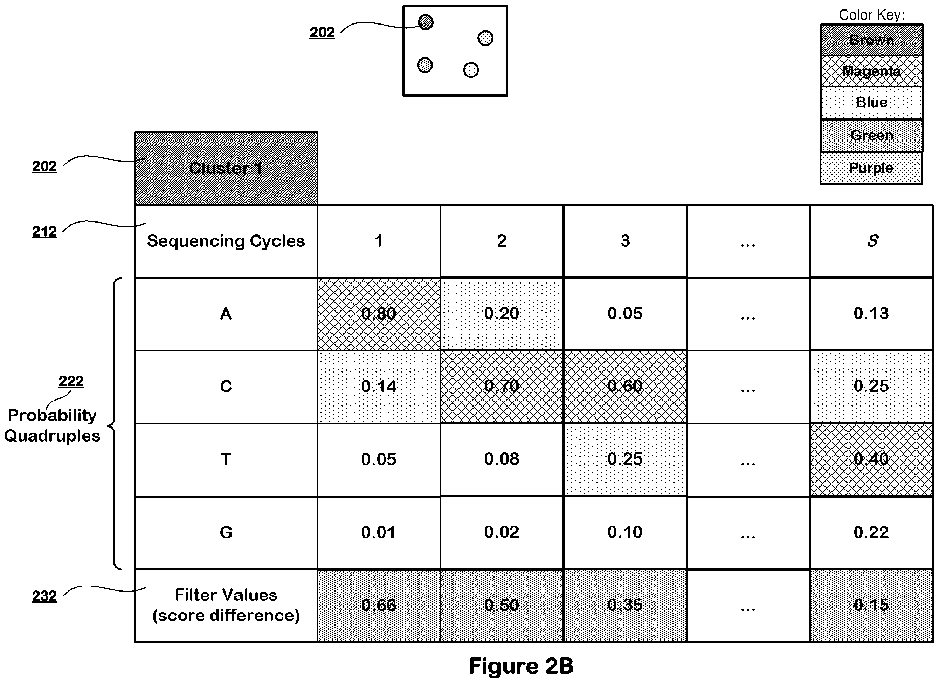

A particular per-cluster, per-cycle probability quadruple identifies probabilities of a base incorporated in a particular cluster at a particular sequencing cycle being A, C, T, and G. When the output layer of the neural network-based base caller 104 uses a softmax function, the probabilities in the per-cluster, per-cycle probability quadruple are exponentially normalized classification scores that sum to unity. Figure 2B depicts example per-cluster, per-cycle probability quadruples 222 produced by the softmax function for cluster 1 (202, shown in brown color) and for sequencing cycles 1 through S (212), respectively. In other words, the first subset of sequencing cycles includes S sequencing cycles.

The detection and filtering logic 146 identifies unreliable clusters based on generating filter values from the per-cluster, per-cycle probability quadruple. In this application, the per-cluster, per-cycle probability quadruples are also referred to as base call classification scores or normalized base call classification scores or initial base call classification scores or normalized initial base call classification scores or initial base calls.

A filter calculator 116 determines a filter value for each per-cluster, per-cycle probability quadruple based on the probabilities it identifies, thereby generating a sequence of filter values 232 for each cluster. The sequence of filter values 232 is stored as filter values 126.

The filter value for a per-cluster, per-cycle probability quadruple is determined based on an arithmetic operation involving one or more of the probabilities. In one implementation, the arithmetic operation used by the filter calculator 116 is subtraction. For example, in the implementation illustrated in Figure 2B, the filter value for the per-cluster, per-cycle probability quadruple is determined by subtracting a second highest one of the probabilities (shown in blue color) from a highest one of the probabilities (shown in magenta color).

In another implementation, the arithmetic operation used by the filter calculator 116 is division. For example, the filter value for the per-cluster, per-cycle probability quadruple is determined as a ratio of the highest one of the probabilities (shown in magenta color) to the second highest one of the probabilities (shown in blue color). In yet another implementation, the arithmetic operation used by the filter calculator 116 is addition. In yet further implementation, the arithmetic operation used by the filter calculator 116 is multiplication.

In one implementation, the filter calculator 116 generates the filter values 126 using a filtering function. In one example, the filtering function is a chastity filter that defines chastity as a ratio of a brightest base intensity divided by a sum of the brightest base intensity and a second brightest base intensity. In another example, the filtering function is at least one of a maximum log probability function, a minimum squared error function, average signal-to-noise ratio (SNR), and a minimum absolute error function.

The unreliable cluster identifier 136 uses the filter values 126 to identify some clusters in the plurality of clusters as unreliable clusters 124. Data identifying the unreliable clusters 124 can be in computer readable format or medium. The unreliable clusters can be identified by instrument ID, the run number on the instrument, the flow cell ID, the lane number, the tile number, the X coordinate of the cluster, the Y coordinate of the cluster, and unique molecular identifiers (UMIs). The unreliable cluster identifier 136 identifies those clusters in the plurality of clusters as unreliable clusters 124 whose sequences of filter values contain "N" number of filter values below a threshold "M". In one implementation, the "N" ranges from 1 to 5. In another implementation, the "M" ranges from 0.5 to 0.99.

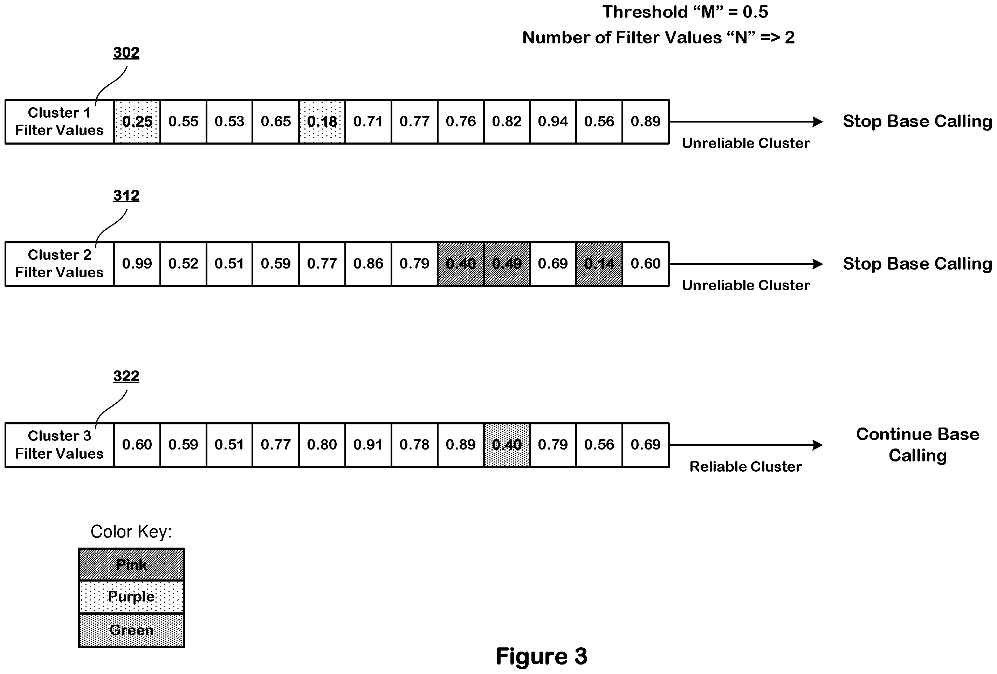

Figure 3 shows an example of identifying the unreliable clusters 124 using the filter values 126. In Figure 3, the threshold "M" is 0.5 and the number of filter values "N" is 2. Figure 3 shows three sequences of filter values 302, 312, and 322 for three clusters 1, 2, and 3, respectively. In the first sequence 302 of cluster 1, there are two filter values below M (shown in purple color), i.e., N = 2, and therefore cluster 1 is identified as an unreliable cluster. In the second sequence 312 of cluster 2, there are three filter values below M (shown in pink color), i.e., N = 3, and therefore cluster 2 is identified as an unreliable cluster. In the third sequence 322 of cluster 3, there is only one filter value below M (shown in green color), i.e., N = 1, and therefore cluster 3 is identified as a reliable cluster.

The discussion now turns to the bypassing logic 142 implemented by the data provider 102. The bypassing logic 142 bypasses base calling the unreliable clusters (e.g., clusters 1 and 2) at a remainder of sequencing cycles of the sequencing run, thereby base calling, at the remainder of sequencing cycles, only those clusters in the plurality of clusters that are not identified as the unreliable clusters. Consider, for example, that the first subset of sequencing cycles of a sequencing run includes 25 sequencing cycles, and the sequencing run has 100 sequencing cycles in total. Then, after the first 25 sequencing cycles, each of the clusters 1, 2, and 3 has a respective sequence of 25 filter values based on the filtering functions described above.

Then, the remainder of sequencing cycles includes the last 75 cycles of the 100-cycle sequencing run. Then, after the first 25 sequencing cycles and before the 26th sequencing cycle, the unreliable cluster identifier 136 determines which of the clusters 1, 2, and 3 are unreliable clusters based on their respective sequences of 25 filter values. Then, at the remainder sequencing cycles, i.e., the last 75 cycles of the 100-cycle sequencing run, the bypassing logic 142 does not base call (i.e., stops base calling) those clusters that are identified as unreliable clusters by the unreliable cluster identifier 136 (e.g., clusters 1 and 2), but continues base calling only those clusters that are not identified as unreliable clusters by the unreliable cluster identifier 136 (e.g., cluster 3). In other words, the unreliable clusters are base called only for cycles 1-25 of the sequencing run and not for cycles 26-100 of the sequencing run, but the reliable clusters are base called for all the cycles 1-100 of the sequencing run.

The term filtering as used in relation to clusters and base calling refers to discarding or disregarding the cluster as a data point. Thus, any clusters of poor intensity or quality can be filtered and are not included in an output data set. In some implementations, filtering of low-quality clusters takes place at one or more discrete points during a sequencing run. In some implementations, filtering occurs during template generation. Alternatively, or additionally, in some implementations, filtering occurs after a predefined cycle. In certain implementations, filtering occurs at or after cycle 1, 2, 3, 4, 5, 6, 7, 8, 9, 10, 11, 12, 13, 14, 15, 16, 17, 18, 19, 20, 21, 22, 23, 24, 25, 26, 27, 28, 29, or after cycle 30 or later. In some implementations, filtering occurs at cycle 25, such that clusters that are not reliable based on the sequence of filter values determined for the first 25 cycles are filtered out.

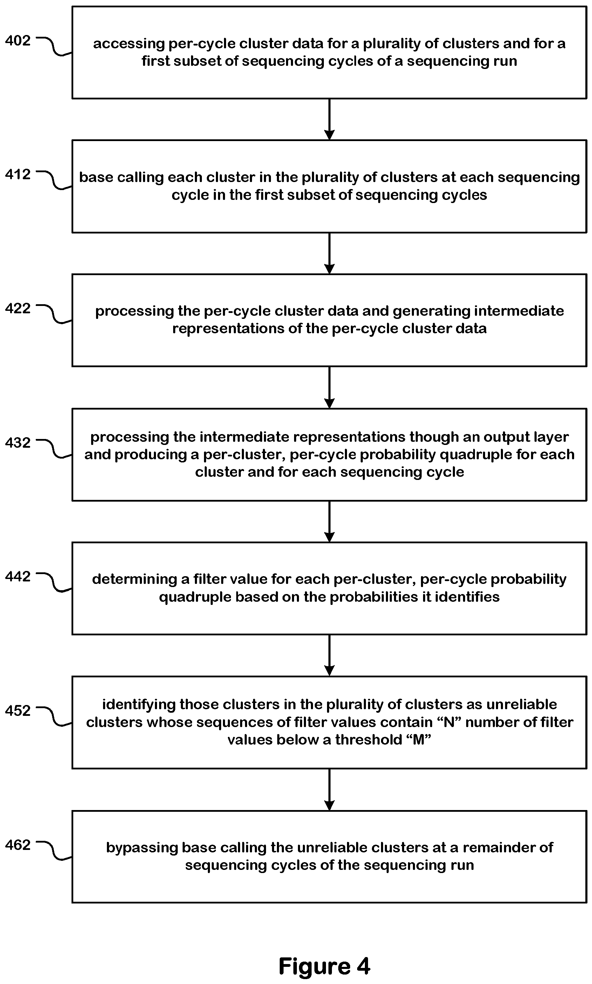

Figure 4 is a flowchart illustrating one implementation of a method of identifying unreliable clusters to improve accuracy and efficiency of base calling. Various processes and steps of the methods set forth herein can be carried out using a computer. The computer can include a processor that is part of a detection device, networked with a detection device used to obtain the data that is processed by the computer or separate from the detection device. In some implementations, information (e.g., image data) may be transmitted between components of a system disclosed herein directly or via a computer network. A local area network (LAN) or wide area network (WAN) may be a corporate computing network, including access to the Internet, to which computers and computing devices comprising the system are connected. In one implementation, the LAN conforms to the transmission control protocol/internet protocol (TCP/IP) industry standard. In some instances, the information (e.g., image data) is input to a system disclosed herein via an input device (e.g., disk drive, compact disk player, USB port etc.). In some instances, the information is received by loading the information, e.g., from a storage device such as a disk or flash drive.

A processor that is used to run an algorithm or other process set forth herein may comprise a microprocessor. The microprocessor may be any conventional general purpose single- or multi-chip microprocessor such as a Pentium™ processor made by Intel Corporation. A particularly useful computer can utilize an Intel Ivybridge dual-12 core processor, LSI raid controller, having 128 GB of RAM, and 2 TB solid state disk drive. In addition, the processor may comprise any conventional special purpose processor such as a digital signal processor or a graphics processor. The processor typically has conventional address lines, conventional data lines, and one or more conventional control lines.

The implementations disclosed herein may be implemented as a method, apparatus, system or article of manufacture using standard programming or engineering techniques to produce software, firmware, hardware, or any combination thereof. The term "article of manufacture" as used herein refers to code or logic implemented in hardware or computer readable media such as optical storage devices, and volatile or non-volatile memory devices. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices. In particular implementations, information or algorithms set forth herein are present in non-transient storage media.

In particular implementations, a computer-implemented method set forth herein can occur in real time while multiple images of an object are being obtained. Such real time analysis is particularly useful for nucleic acid sequencing applications wherein an array of nucleic acids is subjected to repeated cycles of fluidic and detection steps. Analysis of the sequencing data can often be computationally intensive such that it can be beneficial to perform the methods set forth herein in real time or in the background while other data acquisition or analysis algorithms are in process. Example real time analysis methods that can be used with the present methods are those used for the MiSeq and HiSeq sequencing devices commercially available from Illumina, Inc. (San Diego, Calif.) and/or described in US Pat. App. Pub. No. 2012/0020537 A1.

At action 402, the method includes accessing per-cycle cluster data for a plurality of clusters and for a first subset of sequencing cycles of a sequencing run.

At action 412, the method includes base calling each cluster in the plurality of clusters at each sequencing cycle in the first subset of sequencing cycles.

At action 422, the method includes processing the per-cycle cluster data and generating intermediate representations of the per-cycle cluster data.

At action 432, the method includes processing the intermediate representations though an output layer and producing a per-cluster, per-cycle probability quadruple for each cluster and for each sequencing cycle. A particular per-cluster, per-cycle probability quadruple identifies probabilities of a base incorporated in a particular cluster at a particular sequencing cycle being A, C, T, and G.

At action 442, the method includes determining a filter value for each per-cluster, per-cycle probability quadruple based on the probabilities it identifies, thereby generating a sequence of filter values for each cluster.

At action 452, the method includes identifying those clusters in the plurality of clusters as unreliable clusters whose sequences of filter values contain at least "N" number of filter values below a threshold "M".

At action 462, the method includes bypassing base calling the unreliable clusters at a remainder of sequencing cycles of the sequencing run, thereby base calling, at the remainder of sequencing cycles, only those clusters in the plurality of clusters that are not identified as the unreliable clusters.

## Sequencing System

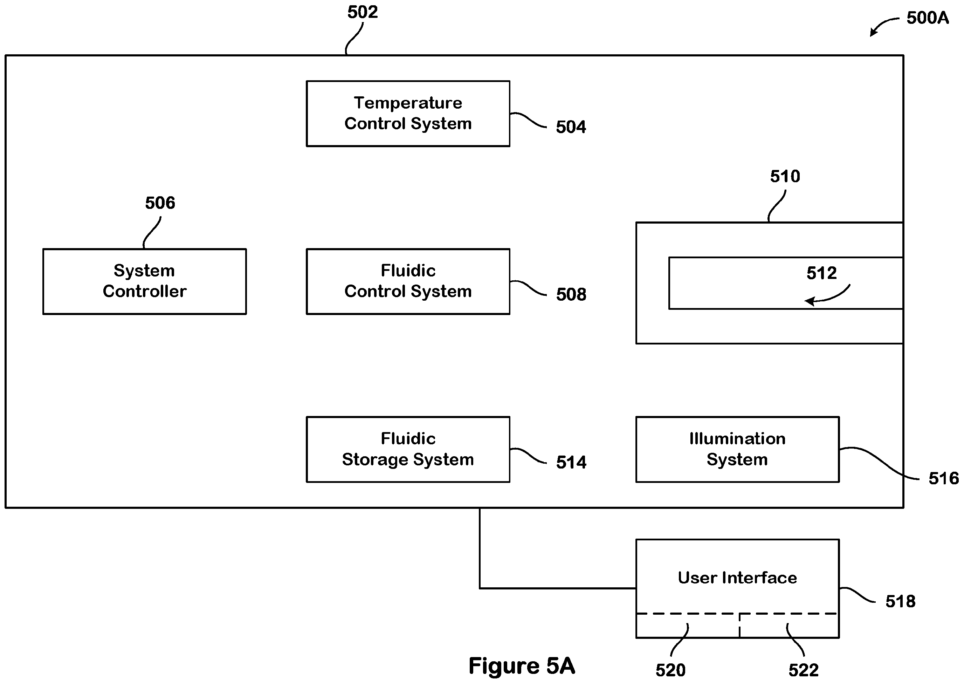

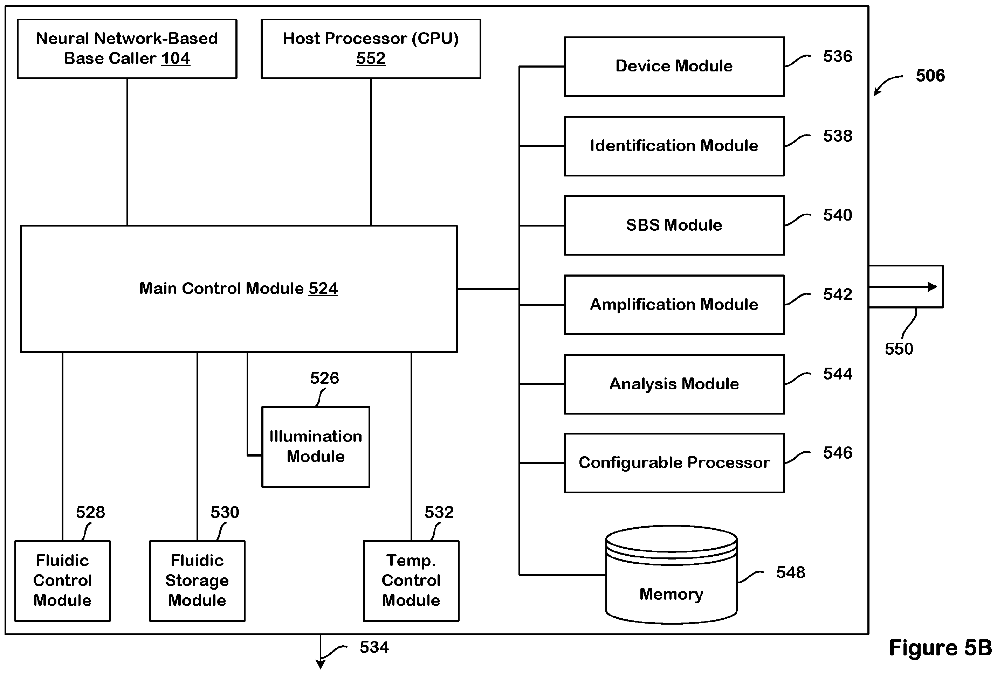

Figures 5A and 5B depict one implementation of a sequencing system 500A. The sequencing system 500A comprises a configurable processor 546. The configurable processor 546 implements the base calling techniques disclosed herein. The sequencing system is also referred to as a "sequencer."

The sequencing system 500A can operate to obtain any information or data that relates to at least one of a biological or chemical substance. In some implementations, the sequencing system 500A is a workstation that may be similar to a bench-top device or desktop computer. For example, a majority (or all) of the systems and components for conducting the desired reactions can be within a common housing 502.

In particular implementations, the sequencing system 500A is a nucleic acid sequencing system configured for various applications, including but not limited to de novo sequencing, resequencing of whole genomes or target genomic regions, and metagenomics. The sequencer may also be used for DNA or RNA analysis. In some implementations, the sequencing system 500A may also be configured to generate reaction sites in a biosensor. For example, the sequencing system 500A may be configured to receive a sample and generate surface attached clusters of clonally amplified nucleic acids derived from the sample. Each cluster may constitute or be part of a reaction site in the biosensor.

The exemplary sequencing system 500A may include a system receptacle or interface 510 that is configured to interact with a biosensor 512 to perform desired reactions within the biosensor 512. In the following description with respect to Figure 5A, the biosensor 512 is loaded into the system receptacle 510. However, it is understood that a cartridge that includes the biosensor 512 may be inserted into the system receptacle 510 and in some states the cartridge can be removed temporarily or permanently. As described above, the cartridge may include, among other things, fluidic control and fluidic storage components.

In particular implementations, the sequencing system 500A is configured to perform a large number of parallel reactions within the biosensor 512. The biosensor 512 includes one or more reaction sites where desired reactions can occur. The reaction sites may be, for example, immobilized to a solid surface of the biosensor or immobilized to beads (or other movable substrates) that are located within corresponding reaction chambers of the biosensor. The reaction sites can include, for example, clusters of clonally amplified nucleic acids. The biosensor 512 may include a solid-state imaging device (e.g., CCD or CMOS imager) and a flow cell mounted thereto. The flow cell may include one or more flow channels that receive a solution from the sequencing system 500A and direct the solution toward the reaction sites. Optionally, the biosensor 512 can be configured to engage a thermal element for transferring thermal energy into or out of the flow channel.

The sequencing system 500A may include various components, assemblies, and systems (or sub-systems) that interact with each other to perform a predetermined method or assay protocol for biological or chemical analysis. For example, the sequencing system 500A includes a system controller 506 that may communicate with the various components, assemblies, and sub-systems of the sequencing system 500A and also the biosensor 512. For example, in addition to the system receptacle 510, the sequencing system 500A may also include a fluidic control system 508 to control the flow of fluid throughout a fluid network of the sequencing system 500A and the biosensor 512; a fluid storage system 514 that is configured to hold all fluids (e.g., gas or liquids) that may be used by the bioassay system; a temperature control system 504 that may regulate the temperature of the fluid in the fluid network, the fluid storage system 514, and/or the biosensor 512; and an illumination system 516 that is configured to illuminate the biosensor 512. As described above, if a cartridge having the biosensor 512 is loaded into the system receptacle 510, the cartridge may also include fluidic control and fluidic storage components.

Also shown, the sequencing system 500A may include a user interface 518 that interacts with the user. For example, the user interface 518 may include a display 520 to display or request information from a user and a user input device 522 to receive user inputs. In some implementations, the display 520 and the user input device 522 are the same device. For example, the user interface 518 may include a touch-sensitive display configured to detect the presence of an individual's touch and also identify a location of the touch on the display. However, other user input devices 522 may be used, such as a mouse, touchpad, keyboard, keypad, handheld scanner, voice-recognition system, motion-recognition system, and the like. As will be discussed in greater detail below, the sequencing system 500A may communicate with various components, including the biosensor 512 (e.g., in the form of a cartridge), to perform the desired reactions. The sequencing system 500A may also be configured to analyze data obtained from the biosensor to provide a user with desired information.

The system controller 506 may include any processor-based or microprocessor-based system, including systems using microcontrollers, reduced instruction set computers (RISC), application specific integrated circuits (ASICs), field programmable gate array (FPGAs), coarse-grained reconfigurable architectures (CGRAs), logic circuits, and any other circuit or processor capable of executing functions described herein. The above examples are exemplary only, and are thus not intended to limit in any way the definition and/or meaning of the term system controller. In the exemplary implementation, the system controller 506 executes a set of instructions that are stored in one or more storage elements, memories, or modules in order to at least one of obtain and analyze detection data. Detection data can include a plurality of sequences of pixel signals, such that a sequence of pixel signals from each of the millions of sensors (or pixels) can be detected over many base calling cycles. Storage elements may be in the form of information sources or physical memory elements within the sequencing system 500A.

The set of instructions may include various commands that instruct the sequencing system 500A or biosensor 512 to perform specific operations such as the methods and processes of the various implementations described herein. The set of instructions may be in the form of a software program, which may form part of a tangible, non-transitory computer readable medium or media. As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

The software may be in various forms such as system software or application software. Further, the software may be in the form of a collection of separate programs, or a program module within a larger program or a portion of a program module. The software also may include modular programming in the form of obj ect-oriented programming. After obtaining the detection data, the detection data may be automatically processed by the sequencing system 500A, processed in response to user inputs, or processed in response to a request made by another processing machine (e.g., a remote request through a communication link). In the illustrated implementation, the system controller 506 includes an analysis module 544. In other implementations, system controller 506 does not include the analysis module 544 and instead has access to the analysis module 544 (e.g., the analysis module 544 may be separately hosted on cloud).

The system controller 506 may be connected to the biosensor 512 and the other components of the sequencing system 500A via communication links. The system controller 506 may also be communicatively connected to off-site systems or servers. The communication links may be hardwired, corded, or wireless. The system controller 506 may receive user inputs or commands, from the user interface 518 and the user input device 522.

The fluidic control system 508 includes a fluid network and is configured to direct and regulate the flow of one or more fluids through the fluid network. The fluid network may be in fluid communication with the biosensor 512 and the fluid storage system 514. For example, select fluids may be drawn from the fluid storage system 514 and directed to the biosensor 512 in a controlled manner, or the fluids may be drawn from the biosensor 512 and directed toward, for example, a waste reservoir in the fluid storage system 514. Although not shown, the fluidic control system 508 may include flow sensors that detect a flow rate or pressure of the fluids within the fluid network. The sensors may communicate with the system controller 506.

The temperature control system 504 is configured to regulate the temperature of fluids at different regions of the fluid network, the fluid storage system 514, and/or the biosensor 512. For example, the temperature control system 504 may include a thermocycler that interfaces with the biosensor 512 and controls the temperature of the fluid that flows along the reaction sites in the biosensor 512. The temperature control system 504 may also regulate the temperature of solid elements or components of the sequencing system 500A or the biosensor 512. Although not shown, the temperature control system 504 may include sensors to detect the temperature of the fluid or other components. The sensors may communicate with the system controller 506.

The fluid storage system 514 is in fluid communication with the biosensor 512 and may store various reaction components or reactants that are used to conduct the desired reactions therein. The fluid storage system 514 may also store fluids for washing or cleaning the fluid network and biosensor 512 and for diluting the reactants. For example, the fluid storage system 514 may include various reservoirs to store samples, reagents, enzymes, other biomolecules, buffer solutions, aqueous, and non-polar solutions, and the like. Furthermore, the fluid storage system 514 may also include waste reservoirs for receiving waste products from the biosensor 512. In implementations that include a cartridge, the cartridge may include one or more of a fluid storage system, fluidic control system or temperature control system. Accordingly, one or more of the components set forth herein as relating to those systems can be contained within a cartridge housing. For example, a cartridge can have various reservoirs to store samples, reagents, enzymes, other biomolecules, buffer solutions, aqueous, and non-polar solutions, waste, and the like. As such, one or more of a fluid storage system, fluidic control system or temperature control system can be removably engaged with a bioassay system via a cartridge or other biosensor.

The illumination system 516 may include a light source (e.g., one or more LEDs) and a plurality of optical components to illuminate the biosensor. Examples of light sources may include lasers, arc lamps, LEDs, or laser diodes. The optical components may be, for example, reflectors, dichroics, beam splitters, collimators, lenses, filters, wedges, prisms, mirrors, detectors, and the like. In implementations that use an illumination system, the illumination system 516 may be configured to direct an excitation light to reaction sites. As one example, fluorophores may be excited by green wavelengths of light, as such the wavelength of the excitation light may be approximately 532 nm. In one implementation, the illumination system 516 is configured to produce illumination that is parallel to a surface normal of a surface of the biosensor 512. In another implementation, the illumination system 516 is configured to produce illumination that is off-angle relative to the surface normal of the surface of the biosensor 512. In yet another implementation, the illumination system 516 is configured to produce illumination that has plural angles, including some parallel illumination and some off-angle illumination.

The system receptacle or interface 510 is configured to engage the biosensor 512 in at least one of a mechanical, electrical, and fluidic manner. The system receptacle 510 may hold the biosensor 512 in a desired orientation to facilitate the flow of fluid through the biosensor 512. The system receptacle 510 may also include electrical contacts that are configured to engage the biosensor 512 so that the sequencing system 500A may communicate with the biosensor 512 and/or provide power to the biosensor 512. Furthermore, the system receptacle 510 may include fluidic ports (e.g., nozzles) that are configured to engage the biosensor 512. In some implementations, the biosensor 512 is removably coupled to the system receptacle 510 in a mechanical manner, in an electrical manner, and also in a fluidic manner.

In addition, the sequencing system 500A may communicate remotely with other systems or networks or with other bioassay systems 500A. Detection data obtained by the bioassay system(s) 500A may be stored in a remote database.

Figure 5B is a block diagram of a system controller 506 that can be used in the system of Figure 5A. In one implementation, the system controller 506 includes one or more processors or modules that can communicate with one another. Each of the processors or modules may include an algorithm (e.g., instructions stored on a tangible and/or non-transitory computer readable storage medium) or sub-algorithms to perform particular processes. The system controller 506 is illustrated conceptually as a collection of modules, but may be implemented utilizing any combination of dedicated hardware boards, DSPs, processors, etc. Alternatively, the system controller 506 may be implemented utilizing an off-the-shelf PC with a single processor or multiple processors, with the functional operations distributed between the processors. As a further option, the modules described below may be implemented utilizing a hybrid configuration in which certain modular functions are performed utilizing dedicated hardware, while the remaining modular functions are performed utilizing an off-the-shelf PC and the like. The modules also may be implemented as software modules within a processing unit.

During operation, a communication port 550 may transmit information (e.g., commands) to or receive information (e.g., data) from the biosensor 512 (Figure 5A) and/or the sub-systems 508, 514, 504 (Figure 5A). In implementations, the communication port 550 may output a plurality of sequences of pixel signals. A communication link 534 may receive user input from the user interface 518 (Figure 5A) and transmit data or information to the user interface 518. Data from the biosensor 512 or sub-systems 508, 514, 504 may be processed by the system controller 506 in real-time during a bioassay session. Additionally or alternatively, data may be stored temporarily in a system memory during a bioassay session and processed in slower than real-time or off-line operation.

As shown in Figure 5B, the system controller 506 may include a plurality of modules 526-548 that communicate with a main control module 524, along with a central processing unit (CPU) 552. The main control module 524 may communicate with the user interface 518 (Figure 5A). Although the modules 526-548 are shown as communicating directly with the main control module 524, the modules 526-548 may also communicate directly with each other, the user interface 518, and the biosensor 512. Also, the modules 526-548 may communicate with the main control module 524 through the other modules.

The plurality of modules 526-548 include system modules 528-532, 526 that communicate with the sub-systems 508, 514, 504, and 516, respectively. The fluidic control module 528 may communicate with the fluidic control system 508 to control the valves and flow sensors of the fluid network for controlling the flow of one or more fluids through the fluid network. The fluid storage module 530 may notify the user when fluids are low or when the waste reservoir is at or near capacity. The fluid storage module 530 may also communicate with the temperature control module 532 so that the fluids may be stored at a desired temperature. The illumination module 526 may communicate with the illumination system 516 to illuminate the reaction sites at designated times during a protocol, such as after the desired reactions (e.g., binding events) have occurred. In some implementations, the illumination module 526 may communicate with the illumination system 516 to illuminate the reaction sites at designated angles.

The plurality of modules 526-548 may also include a device module 536 that communicates with the biosensor 512 and an identification module 538 that determines identification information relating to the biosensor 512. The device module 536 may, for example, communicate with the system receptacle 510 to confirm that the biosensor has established an electrical and fluidic connection with the sequencing system 500A. The identification module 538 may receive signals that identify the biosensor 512. The identification module 538 may use the identity of the biosensor 512 to provide other information to the user. For example, the identification module 538 may determine and then display a lot number, a date of manufacture, or a protocol that is recommended to be run with the biosensor 512.

The plurality of modules 526-548 also includes an analysis module 544 (also called signal processing module or signal processor) that receives and analyzes the signal data (e.g., image data) from the biosensor 512. Analysis module 544 includes memory (e.g., RAM or Flash) to store detection/image data. Detection data can include a plurality of sequences of pixel signals, such that a sequence of pixel signals from each of the millions of sensors (or pixels) can be detected over many base calling cycles. The signal data may be stored for subsequent analysis or may be transmitted to the user interface 518 to display desired information to the user. In some implementations, the signal data may be processed by the solid-state imager (e.g., CMOS image sensor) before the analysis module 544 receives the signal data.

The analysis module 544 is configured to obtain image data from the light detectors at each of a plurality of sequencing cycles. The image data is derived from the emission signals detected by the light detectors and process the image data for each of the plurality of sequencing cycles through the neural network-based base caller 104 and produce a base call for at least some of the analytes at each of the plurality of sequencing cycle. The light detectors can be part of one or more over-head cameras (e.g., Illumina's GAIIx's CCD camera taking images of the clusters on the biosensor 512 from the top), or can be part of the biosensor 512 itself (e.g., Illumina's iSeq's CMOS image sensors underlying the clusters on the biosensor 512 and taking images of the clusters from the bottom).

The output of the light detectors is the sequencing images, each depicting intensity emissions of the clusters and their surrounding background. The sequencing images depict intensity emissions generated as a result of nucleotide incorporation in the sequences during the sequencing. The intensity emissions are from associated analytes and their surrounding background. The sequencing images are stored in memory 548.

Protocol modules 540 and 542 communicate with the main control module 524 to control the operation of the sub-systems 508, 514, and 504 when conducting predetermined assay protocols. The protocol modules 540 and 542 may include sets of instructions for instructing the sequencing system 500A to perform specific operations pursuant to predetermined protocols. As shown, the protocol module may be a sequencing-by-synthesis (SBS) module 540 that is configured to issue various commands for performing sequencing-by-synthesis processes. In SBS, extension of a nucleic acid primer along a nucleic acid template is monitored to determine the sequence of nucleotides in the template. The underlying chemical process can be polymerization (e.g., as catalyzed by a polymerase enzyme) or ligation (e.g., catalyzed by a ligase enzyme). In a particular polymerase-based SBS implementation, fluorescently labeled nucleotides are added to a primer (thereby extending the primer) in a template dependent fashion such that detection of the order and type of nucleotides added to the primer can be used to determine the sequence of the template. For example, to initiate a first SBS cycle, commands can be given to deliver one or more labeled nucleotides, DNA polymerase, etc., into/through a flow cell that houses an array of nucleic acid templates. The nucleic acid templates may be located at corresponding reaction sites. Those reaction sites where primer extension causes a labeled nucleotide to be incorporated can be detected through an imaging event. During an imaging event, the illumination system 516 may provide an excitation light to the reaction sites. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for implementations that use reversible termination a command can be given to deliver a deblocking reagent to the flow cell (before or after detection occurs). One or more commands can be given to effect wash(es) between the various delivery steps. The cycle can then be repeated n times to extend the primer by n nucleotides, thereby detecting a sequence of length n. Exemplary sequencing techniques are described, for example, in Bentley et al., Nature 456:53-59 (2005); WO 04/015497; US 7,057,026; WO 91/06675; WO 07/123744; US 7,329,492; US 7,211,414; US 7,315,019; US 7,405,251, and US 2005/014705052,

For the nucleotide delivery step of an SBS cycle, either a single type of nucleotide can be delivered at a time, or multiple different nucleotide types (e.g., A, C, T and G together) can be delivered. For a nucleotide delivery configuration where only a single type of nucleotide is present at a time, the different nucleotides need not have distinct labels since they can be distinguished based on temporal separation inherent in the individualized delivery. Accordingly, a sequencing method or apparatus can use single color detection. For example, an excitation source need only provide excitation at a single wavelength or in a single range of wavelengths. For a nucleotide delivery configuration where delivery results in multiple different nucleotides being present in the flow cell at one time, sites that incorporate different nucleotide types can be distinguished based on different fluorescent labels that are attached to respective nucleotide types in the mixture. For example, four different nucleotides can be used, each having one of four different fluorophores. In one implementation, the four different fluorophores can be distinguished using excitation in four different regions of the spectrum. For example, four different excitation radiation sources can be used. Alternatively, fewer than four different excitation sources can be used, but optical filtration of the excitation radiation from a single source can be used to produce different ranges of excitation radiation at the flow cell.

In some implementations, fewer than four different colors can be detected in a mixture having four different nucleotides. For example, pairs of nucleotides can be detected at the same wavelength, but distinguished based on a difference in intensity for one member of the pair compared to the other, or based on a change to one member of the pair (e.g., via chemical modification, photochemical modification or physical modification) that causes apparent signal to appear or disappear compared to the signal detected for the other member of the pair. Exemplary apparatus and methods for distinguishing four different nucleotides using detection of fewer than four colors are described for example in US Pat. App. Ser. Nos. 61/535,294 and 61/619,575. U.S. Application No 13/624,200, which was filed on September 21, 2012.

The plurality of protocol modules may also include a sample-preparation (or generation) module 542 that is configured to issue commands to the fluidic control system 508 and the temperature control system 504 for amplifying a product within the biosensor 512. For example, the biosensor 512 may be engaged to the sequencing system 500A. The amplification module 542 may issue instructions to the fluidic control system 508 to deliver necessary amplification components to reaction chambers within the biosensor 512. In other implementations, the reaction sites may already contain some components for amplification, such as the template DNA and/or primers. After delivering the amplification components to the reaction chambers, the amplification module 542 may instruct the temperature control system 504 to cycle through different temperature stages according to known amplification protocols. In some implementations, the amplification and/or nucleotide incorporation is performed isothermally.

The SBS module 540 may issue commands to perform bridge PCR where clusters of clonal amplicons are formed on localized areas within a channel of a flow cell. After generating the amplicons through bridge PCR, the amplicons may be "linearized" to make single stranded template DNA, or sstDNA, and a sequencing primer may be hybridized to a universal sequence that flanks a region of interest. For example, a reversible terminator-based sequencing by synthesis method can be used as set forth above or as follows.

Each base calling or sequencing cycle can extend an sstDNA by a single base which can be accomplished for example by using a modified DNA polymerase and a mixture of four types of nucleotides. The different types of nucleotides can have unique fluorescent labels, and each nucleotide can further have a reversible terminator that allows only a single-base incorporation to occur in each cycle. After a single base is added to the sstDNA, excitation light may be incident upon the reaction sites and fluorescent emissions may be detected. After detection, the fluorescent label and the terminator may be chemically cleaved from the sstDNA. Another similar base calling or sequencing cycle may follow. In such a sequencing protocol, the SBS module 540 may instruct the fluidic control system 508 to direct a flow of reagent and enzyme solutions through the biosensor 512. Exemplary reversible terminator-based SBS methods which can be utilized with the apparatus and methods set forth herein are described in US Patent Application Publication No. 2007/0166705 A1, US Patent Application Publication No. 2006/01563901 A1, US Patent No. 7,057,026, US Patent Application Publication No. 2006/0240439 A1, US Patent Application Publication No. 2006/02514714709 A1, PCT Publication No. WO 05/065514, US Patent Application Publication No. 2005/014700900 A1, PCT Publication No. WO 06/05B199 and PCT Publication No. WO 07/01470251. Exemplary reagents for reversible terminator-based SBS are described in US 7,541,444; US 7,057,026; US 7,414,14716; US 7,427,673; US 7,566,537; US 7,592,435 and WO 07/14535365.

In some implementations, the amplification and SBS modules may operate in a single assay protocol where, for example, template nucleic acid is amplified and subsequently sequenced within the same cartridge.

The sequencing system 500A may also allow the user to reconfigure an assay protocol. For example, the sequencing system 500A may offer options to the user through the user interface 518 for modifying the determined protocol. For example, if it is determined that the biosensor 512 is to be used for amplification, the sequencing system 500A may request a temperature for the annealing cycle. Furthermore, the sequencing system 500A may issue warnings to a user if a user has provided user inputs that are generally not acceptable for the selected assay protocol.

In implementations, the biosensor 512 includes millions of sensors (or pixels), each of which generates a plurality of sequences of pixel signals over successive base calling cycles. The analysis module 544 detects the plurality of sequences of pixel signals and attributes them to corresponding sensors (or pixels) in accordance to the row-wise and/or column-wise location of the sensors on an array of sensors.

## Configurable Processor

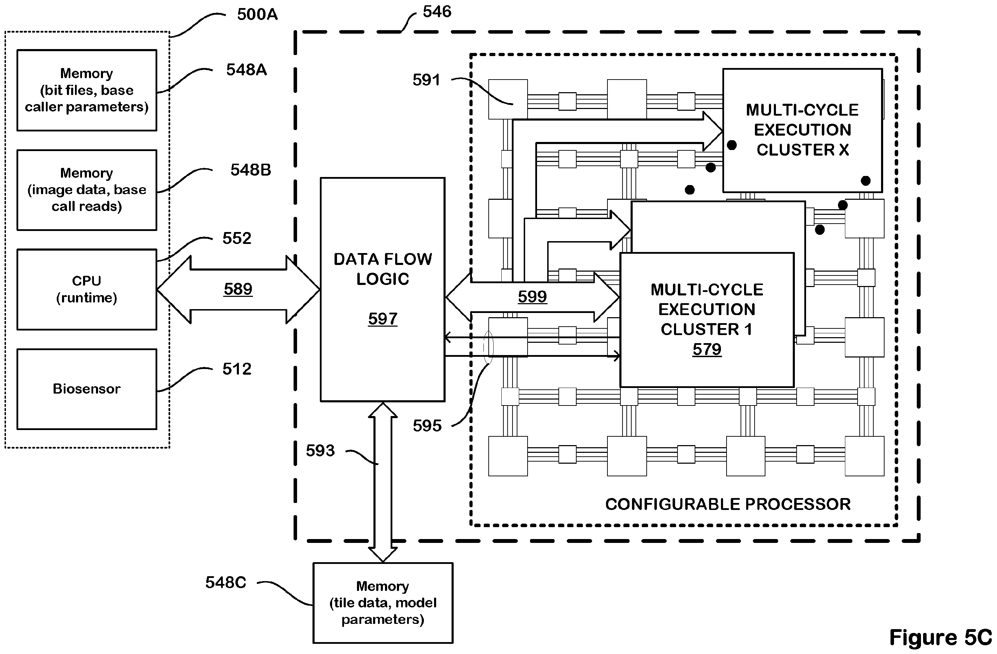

Figure 5C is a simplified block diagram of a system for analysis of sensor data from the sequencing system 500A, such as base call sensor outputs. In the example of Figure 5C, the system includes the configurable processor 546. The configurable processor 546 can execute a base caller (e.g., the neural network-based base caller 104) in coordination with a runtime program executed by the central processing unit (CPU) 552 (i.e., a host processor). The sequencing system 500A comprises the biosensor 512 and flow cells. The flow cells can comprise one or more tiles in which clusters of genetic material are exposed to a sequence of analyte flows used to cause reactions in the clusters to identify the bases in the genetic material. The sensors sense the reactions for each cycle of the sequence in each tile of the flow cell to provide tile data. Genetic sequencing is a data intensive operation, which translates base call sensor data into sequences of base calls for each cluster of genetic material sensed in during a base call operation.

The system in this example includes the CPU 552, which executes a runtime program to coordinate the base call operations, memory 548B to store sequences of arrays of tile data, base call reads produced by the base calling operation, and other information used in the base call operations. Also, in this illustration the system includes memory 548A to store a configuration file (or files), such as FPGA bit files, and model parameters for the neural networks used to configure and reconfigure the configurable processor 546, and execute the neural networks. The sequencing system 500A can include a program for configuring a configurable processor and in some implementations a reconfigurable processor to execute the neural networks.

The sequencing system 500A is coupled by a bus 589 to the configurable processor 546. The bus 589 can be implemented using a high throughput technology, such as in one example bus technology compatible with the PCIe standards (Peripheral Component Interconnect Express) currently maintained and developed by the PCI-SIG (PCI Special Interest Group). Also in this example, a memory 548A is coupled to the configurable processor 546 by bus 593. The memory 548A can be on-board memory, disposed on a circuit board with the configurable processor 546. The memory 548A is used for high speed access by the configurable processor 546 of working data used in the base call operation. The bus 593 can also be implemented using a high throughput technology, such as bus technology compatible with the PCIe standards.

Configurable processors, including field programmable gate arrays FPGAs, coarse grained reconfigurable arrays CGRAs, and other configurable and reconfigurable devices, can be configured to implement a variety of functions more efficiently or faster than might be achieved using a general purpose processor executing a computer program. Configuration of configurable processors involves compiling a functional description to produce a configuration file, referred to sometimes as a bitstream or bit file, and distributing the configuration file to the configurable elements on the processor. The configuration file defines the logic functions to be executed by the configurable processor, by configuring the circuit to set data flow patterns, use of distributed memory and other on-chip memory resources, lookup table contents, operations of configurable logic blocks and configurable execution units like multiply-and-accumulate units, configurable interconnects and other elements of the configurable array. A configurable processor is reconfigurable if the configuration file may be changed in the field, by changing the loaded configuration file. For example, the configuration file may be stored in volatile SRAM elements, in non-volatile read-write memory elements, and in combinations of the same, distributed among the array of configurable elements on the configurable or reconfigurable processor. A variety of commercially available configurable processors are suitable for use in a base calling operation as described herein. Examples include Google's Tensor Processing Unit (TPU)™, rackmount solutions like GX4 Rackmount Series™, GX9 Rackmount Series™, NVIDIA DGX-1™, Microsoft' Stratix V FPGA™, Graphcore's Intelligent Processor Unit (IPU)™, Qualcomm's Zeroth Platform™ with Snapdragon processors™, NVIDIA's Volta™, NVIDIA's DRIVE PX™, NVIDIA's JETSON TX1/TX2 MODULE™, Intel's Nirvana™, Movidius VPU™, Fujitsu DPI™, ARM's DynamicIQ™, IBM TrueNorth™, Lambda GPU Server with Testa V100s™, Xilinx Alveo™ U200, Xilinx Alveo™ U250, Xilinx Alveo™ U280, Intel/Altera Stratix™ GX2800, Intel/Altera Stratix™ GX2800, and Intel Stratix™ GX10M. In some examples, a host CPU can be implemented on the same integrated circuit as the configurable processor.

Implementations described herein implement the neural network-based base caller 104 using the configurable processor 546. The configuration file for the configurable processor 546 can be implemented by specifying the logic functions to be executed using a high level description language HDL or a register transfer level RTL language specification. The specification can be compiled using the resources designed for the selected configurable processor to generate the configuration file. The same or similar specification can be compiled for the purposes of generating a design for an application-specific integrated circuit which may not be a configurable processor.

Alternatives for the configurable processor configurable processor 546, in all implementations described herein, therefore include a configured processor comprising an application specific ASIC or special purpose integrated circuit or set of integrated circuits, or a system-on-a-chip SOC device, or a graphics processing unit (GPU) processor or a coarse-grained reconfigurable architecture (CGRA) processor, configured to execute a neural network based base call operation as described herein.

In general, configurable processors and configured processors described herein, as configured to execute runs of a neural network, are referred to herein as neural network processors.

The configurable processor 546 is configured in this example by a configuration file loaded using a program executed by the CPU 552, or by other sources, which configures the array of configurable elements 591 (e.g., configuration logic blocks (CLB) such as look up tables (LUTs), flip-flops, compute processing units (PMUs), and compute memory units (CMUs), configurable I/O blocks, programmable interconnects), on the configurable processor to execute the base call function. In this example, the configuration includes data flow logic 597 which is coupled to the buses 589 and 593 and executes functions for distributing data and control parameters among the elements used in the base call operation.

Also, the configurable processor 546 is configured with data flow logic 597 to execute the neural network-based base caller 104. The logic 597 comprises multi-cycle execution clusters (e.g., 579) which, in this example, includes execution cluster 1 through execution cluster X. The number of multi-cycle execution clusters can be selected according to a trade-off involving the desired throughput of the operation, and the available resources on the configurable processor 546.

The multi-cycle execution clusters are coupled to the data flow logic 597 by data flow paths 599 implemented using configurable interconnect and memory resources on the configurable processor 546. Also, the multi-cycle execution clusters are coupled to the data flow logic 597 by control paths 595 implemented using configurable interconnect and memory resources for example on the configurable processor 546, which provide control signals indicating available execution clusters, readiness to provide input units for execution of a run of the neural network-based base caller 104 to the available execution clusters, readiness to provide trained parameters for the neural network-based base caller 104, readiness to provide output patches of base call classification data, and other control data used for execution of the neural network-based base caller 104.

The configurable processor 546 is configured to execute runs of the neural network-based base caller 104 using trained parameters to produce classification data for the sensing cycles of the base calling operation. A run of the neural network-based base caller 104 is executed to produce classification data for a subject sensing cycle of the base calling operation. A run of the neural network-based base caller 104 operates on a sequence including a number N of arrays of tile data from respective sensing cycles of N sensing cycles, where the N sensing cycles provide sensor data for different base call operations for one base position per operation in time sequence in the examples described herein. Optionally, some of the N sensing cycles can be out of sequence if needed according to a particular neural network model being executed. The number N can be any number greater than one. In some examples described herein, sensing cycles of the N sensing cycles represent a set of sensing cycles for at least one sensing cycle preceding the subject sensing cycle and at least one sensing cycle following the subject cycle in time sequence. Examples are described herein in which the number N is an integer equal to or greater than five.

The data flow logic 597 is configured to move tile data and at least some trained parameters of the model parameters from the memory 548A to the configurable processor 546 for runs of the neural network-based base caller 104, using input units for a given run including tile data for spatially aligned patches of the N arrays. The input units can be moved by direct memory access operations in one DMA operation, or in smaller units moved during available time slots in coordination with the execution of the neural network deployed.

Tile data for a sensing cycle as described herein can comprise an array of sensor data having one or more features. For example, the sensor data can comprise two images which are analyzed to identify one of four bases at a base position in a genetic sequence of DNA, RNA, or other genetic material. The tile data can also include metadata about the images and the sensors. For example, in implementations of the base calling operation, the tile data can comprise information about alignment of the images with the clusters such as distance from center information indicating the distance of each pixel in the array of sensor data from the center of a cluster of genetic material on the tile.

During execution of the neural network-based base caller 104 as described below, tile data can also include data produced during execution of the neural network-based base caller 104, referred to as intermediate data, which can be reused rather than recomputed during a run of the neural network-based base caller 104. For example, during execution of the neural network-based base caller 104, the data flow logic 597 can write intermediate data to the memory 548A in place of the sensor data for a given patch of an array of tile data. Implementations like this are described in more detail below.

As illustrated, a system is described for analysis of base call sensor output, comprising memory (e.g., 548A) accessible by the runtime program storing tile data including sensor data for a tile from sensing cycles of a base calling operation. Also, the system includes a neural network processor, such as configurable processor 546 having access to the memory. The neural network processor is configured to execute runs of a neural network using trained parameters to produce classification data for sensing cycles. As described herein, a run of the neural network is operating on a sequence of N arrays of tile data from respective sensing cycles of N sensing cycles, including a subject cycle, to produce the classification data for the subject cycle. The data flow logic 908 is provided to move tile data and the trained parameters from the memory to the neural network processor for runs of the neural network using input units including data for spatially aligned patches of the N arrays from respective sensing cycles of N sensing cycles.

Also, a system is described in which the neural network processor has access to the memory, and includes a plurality of execution clusters, the execution clusters in the plurality of execution clusters configured to execute a neural network. The data flow logic 597 has access to the memory and to execution clusters in the plurality of execution clusters, to provide input units of tile data to available execution clusters in the plurality of execution clusters, the input units including a number N of spatially aligned patches of arrays of tile data from respective sensing cycles, including a subject sensing cycle, and to cause the execution clusters to apply the N spatially aligned patches to the neural network to produce output patches of classification data for the spatially aligned patch of the subject sensing cycle, where N is greater than 1.

## Data Flow Logic

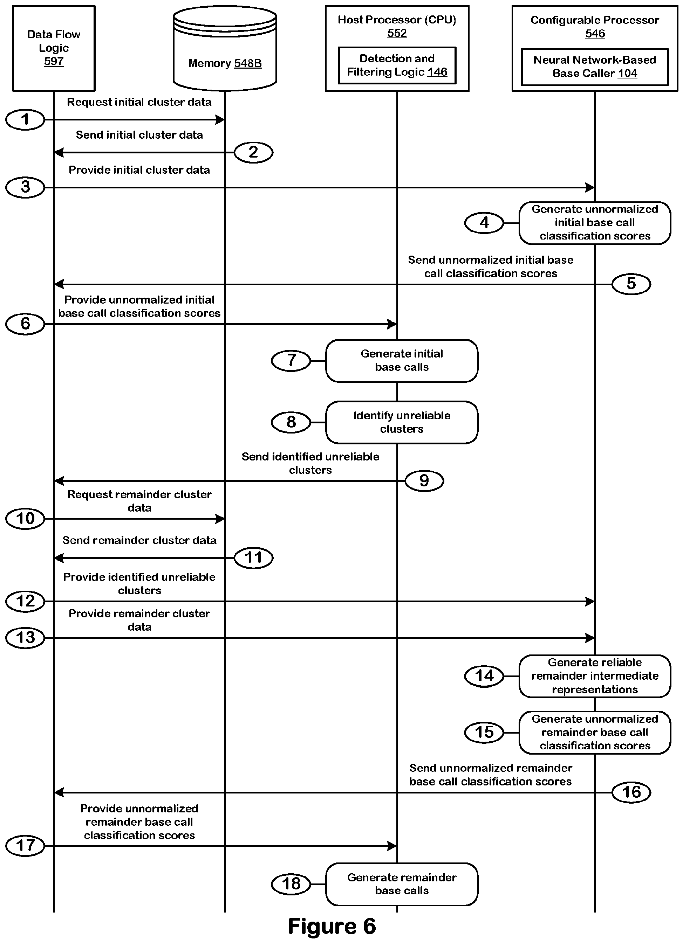

Figure 6 shows one implementation of the disclosed data flow logic that enables a host processor to filter unreliable clusters based on base calls predicted by a neural network running on a configurable processor, and further enables the configurable processor to use data identifying the unreliable clusters to generate reliable remainder intermediate representations.

At action 1, the data flow logic 597 requests initial cluster data from the memory 548B. Initial cluster data includes sequencing images that depict intensity emissions of clusters at initial sequencing cycles of a sequencing run, i.e., a first subset of sequencing cycles of the sequencing run, as discussed above. For example, the initial cluster data can include sequencing images for the first 25 sequencing cycles (initial sequencing cycles) of the sequencing run.

Note that because clusters are arranged on the flow cell at high spatial density (e.g., at low-micron or sub-micron resolution), the sequencing images in the initial cluster data depict intensity emissions from a plurality of clusters that can include both reliable and unreliable clusters. That is, when certain unreliable clusters are adjacent to certain reliable clusters, then the corresponding sequencing images in the initial cluster data depict intensity emissions from both the unreliable clusters and the reliable clusters because the sequencing images in the initial cluster data are captured at an optical resolution that captures light or signal emitted from a plurality of clusters.

At action 2, the memory 548B sends the initial cluster data to the data flow logic 597.

At action 3, the data flow logic 597 provides the initial cluster data to the configurable processor 546.

At action 4, the neural network-based base caller 104, running on the configurable processor 546, generates initial intermediate representations (e.g., feature maps) from the initial cluster data (e.g., by processing the initial cluster data through its spatial and temporal convolution layers), and produces initial base call classification scores for the plurality of clusters and for the initial sequencing cycles based on the initial intermediate representations. In one implementation, the initial base call classification scores are unnormalized, for example, they are not subjected to exponential normalization by a softmax function.

At action 5, the configurable processor 546 sends the unnormalized initial base call classification scores to the data flow logic 597.

At action 6, the data flow logic 597 provides the unnormalized initial base call classification scores to the host processor 552.

At action 7, the host processor 552 normalizes the unnormalized initial base call classification scores (e.g., by applying the softmax function), and generates normalized initial base call classification scores, i.e., initial base calls.

At action 8, the detection and filtering logic 146, running on the host processor 552, uses the normalized initial base call classification scores/initial base calls to identify unreliable clusters in the plurality of clusters based on generating filter values, as discussed above in the section titled "Detecting and Filtering Unreliable Clusters".

At action 9, the host processor 552 sends data identifying the unreliable clusters to the data flow logic 597. The unreliable clusters can be identified by instrument ID, the run number on the instrument, the flow cell ID, the lane number, the tile number, the X coordinate of the cluster, the Y coordinate of the cluster, and unique molecular identifiers (UMIs).

At action 10, the data flow logic 597 requests remainder cluster data from the memory 548B. Remainder cluster data includes sequencing images that depict intensity emissions of clusters at remainder sequencing cycles of the sequencing run, i.e., those sequencing cycles of the sequencing run that do not include the first subset of sequencing cycles of the sequencing run, as discussed above. For example, the remainder cluster data can include sequencing images for the 26 to 100 sequencing cycles (the last 75 sequencing cycles) of a 100-cycle sequencing run.

Note that because clusters are arranged on the flow cell at high spatial density (e.g., at low-micron or sub-micron resolution), the sequencing images in the remainder cluster data depict intensity emissions from a plurality of clusters that can include both reliable and unreliable clusters. That is, when certain unreliable clusters are adjacent to certain reliable clusters, then the corresponding sequencing images in the remainder cluster data depict intensity emissions from both the unreliable clusters and the reliable clusters because the sequencing images in the remainder cluster data are captured at an optical resolution that captures light or signal emitted from a plurality of clusters.

At action 11, the memory 548B sends the remainder cluster data to the data flow logic 597.

At action 12, the data flow logic 597 sends data identifying the unreliable clusters to the configurable processor 546. The unreliable clusters can be identified by instrument ID, the run number on the instrument, the flow cell ID, the lane number, the tile number, the X coordinate of the cluster, the Y coordinate of the cluster, and unique molecular identifiers (UMIs).

At action 13, the data flow logic 597 sends the remainder cluster data to the configurable processor 546.

At action 14, the neural network-based base caller 104, running on the configurable processor 546, generates remainder intermediate representations (e.g., feature maps) from the remainder cluster data (e.g., by processing the remainder cluster data through its spatial convolution layers). The configurable processor 546 uses the data identifying the unreliable clusters to generate reliable remainder intermediate representations by removing, from the remainder intermediate representations, those portions that result from portions of the remainder cluster data that represent the unreliable clusters. In one implementation, the data identifying the unreliable clusters identifies pixels that depict intensity emissions of the unreliable clusters in the initial cluster data and the remainder cluster data. In some implementations, the configurable processor 546 is further configured to generate the reliable remainder intermediate representations by discarding, from pixelated feature maps generated from the remainder cluster data by the neural network-based base caller 104, those feature map pixels that result from pixels of the remainder cluster data that depict intensity emissions of the unreliable clusters captured for the remainder sequencing cycles.

At action 15, the configurable processor 546 is further configured to provide the reliable remainder intermediate representations to the neural network-based base caller 104 and cause the neural network-based base caller 104 to produce remainder base call classification scores only for those clusters in the plurality of clusters that are not the unreliable clusters and for the remainder sequencing cycles, thereby bypassing production of the remainder base call classification scores for the unreliable clusters. In one implementation, the remainder base call classification scores are unnormalized, for example, they are not subjected to exponential normalization by a softmax function.

At action 16, the configurable processor 546 sends the unnormalized remainder base call classification scores to the data flow logic 597.

At action 17, the data flow logic 597 provides the unnormalized remainder base call classification scores to the host processor 552.

At action 18, the host processor 552 normalizes the unnormalized remainder base call classification scores (e.g., by applying the softmax function), and generates normalized remainder base call classification scores, i.e., remainder base calls.

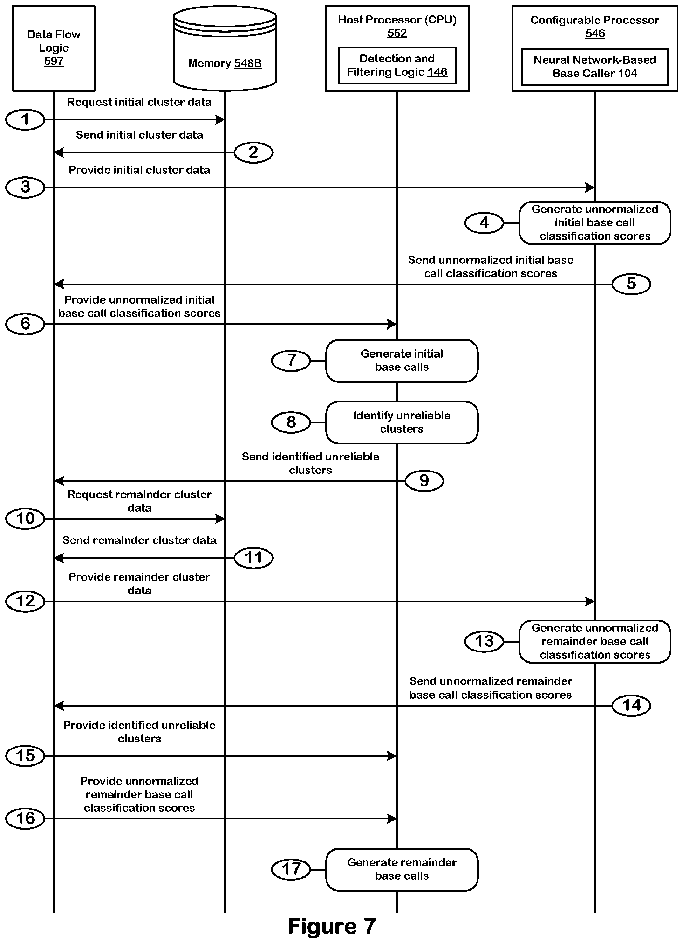

Figure 7 shows another implementation of the disclosed data flow logic that enables the host processor to filter unreliable clusters based on base calls predicted by the neural network running on the configurable processor, and further enables the host processor to use data identifying the unreliable clusters to base call only reliable clusters.

At action 1, the data flow logic 597 requests initial cluster data from the memory 548B. Initial cluster data includes sequencing images that depict intensity emissions of clusters at initial sequencing cycles of a sequencing run, i.e., a first subset of sequencing cycles of the sequencing run, as discussed above. For example, the initial cluster data can include sequencing images for the first 25 sequencing cycles (initial sequencing cycles) of the sequencing run.

Note that because clusters are arranged on the flow cell at high spatial density (e.g., at low-micron or sub-micron resolution), the sequencing images in the initial cluster data depict intensity emissions from a plurality of clusters that can include both reliable and unreliable clusters. That is, when certain unreliable clusters are adjacent to certain reliable clusters, then the corresponding sequencing images in the initial cluster data depict intensity emissions from both the unreliable clusters and the reliable clusters because the sequencing images in the initial cluster data are captured at an optical resolution that captures light or signal emitted from a plurality of clusters.

At action 2, the memory 548B sends the initial cluster data to the data flow logic 597.

At action 3, the data flow logic 597 provides the initial cluster data to the configurable processor 546.

At action 4, the neural network-based base caller 104, running on the configurable processor 546, generates initial intermediate representations (e.g., feature maps) from the initial cluster data (e.g., by processing the initial cluster data through its spatial and temporal convolution layers), and produces initial base call classification scores for the plurality of clusters and for the initial sequencing cycles based on the initial intermediate representations. In one implementation, the initial base call classification scores are unnormalized, for example, they are not subjected to exponential normalization by a softmax function.

At action 5, the configurable processor 546 sends the unnormalized initial base call classification scores to the data flow logic 597.

At action 6, the data flow logic 597 provides the unnormalized initial base call classification scores to the host processor 552.

At action 7, the host processor 552 normalizes the unnormalized initial base call classification scores (e.g., by applying the softmax function), and generates normalized initial base call classification scores, i.e., initial base calls.

At action 8, the detection and filtering logic 146, running on the host processor 552, uses the normalized initial base call classification scores/initial base calls to identify unreliable clusters in the plurality of clusters based on generating filter values, as discussed above in the section titled "Detecting and Filtering Unreliable Clusters".

At action 9, the host processor 552 sends data identifying the unreliable clusters to the data flow logic 597.

At action 10, the data flow logic 597 requests remainder cluster data from the memory 548B. Remainder cluster data includes sequencing images that depict intensity emissions of clusters at remainder sequencing cycles of the sequencing run, i.e., those sequencing cycles of the sequencing run that do not include the first subset of sequencing cycles of the sequencing run, as discussed above. For example, the remainder cluster data can include sequencing images for the 26 to 100 sequencing cycles (the last 75 sequencing cycles) of a 100-cycle sequencing run.

Note that because clusters are arranged on the flow cell at high spatial density (e.g., at low-micron or sub-micron resolution), the sequencing images in the remainder cluster data depict intensity emissions from a plurality of clusters that can include both reliable and unreliable clusters. That is, when certain unreliable clusters are adjacent to certain reliable clusters, then the corresponding sequencing images in the remainder cluster data depict intensity emissions from both the unreliable clusters and the reliable clusters because the sequencing images in the remainder cluster data are captured at an optical resolution that captures light or signal emitted from a plurality of clusters.

At action 11, the memory 548B sends the remainder cluster data to the data flow logic 597.

At action 12, the data flow logic 597 sends the remainder cluster data to the configurable processor 546.

At action 13, the neural network-based base caller 104, running on the configurable processor 546, generates remainder intermediate representations (e.g., feature maps) from the remainder cluster data (e.g., by processing the remainder cluster data through its spatial and temporal convolution layers). The neural network-based base caller 104 further produces remainder base call classification scores for the plurality of clusters and for the remainder sequencing cycles based on the remainder intermediate representations. In one implementation, the remainder base call classification scores are unnormalized, for example, they are not subjected to exponential normalization by a softmax function.

At action 14, the configurable processor 546 sends the unnormalized remainder base call classification scores to the data flow logic 597.

At action 15, the data flow logic 597 sends data identifying the unreliable clusters to the host processor 552.

At action 16, the data flow logic 597 provides the unnormalized remainder base call classification scores to the host processor 552.

At action 17, the host processor 552 normalizes the unnormalized remainder base call classification scores (e.g., by applying the softmax function), and generates normalized remainder base call classification scores, i.e., remainder base calls by using data identifying the unreliable clusters to base call only those clusters in the plurality of clusters that are not the unreliable clusters, thereby bypasses base calling the unreliable clusters at the remainder sequencing cycles. In one implementation, the data identifying the unreliable clusters identifies location coordinates of the unreliable clusters.

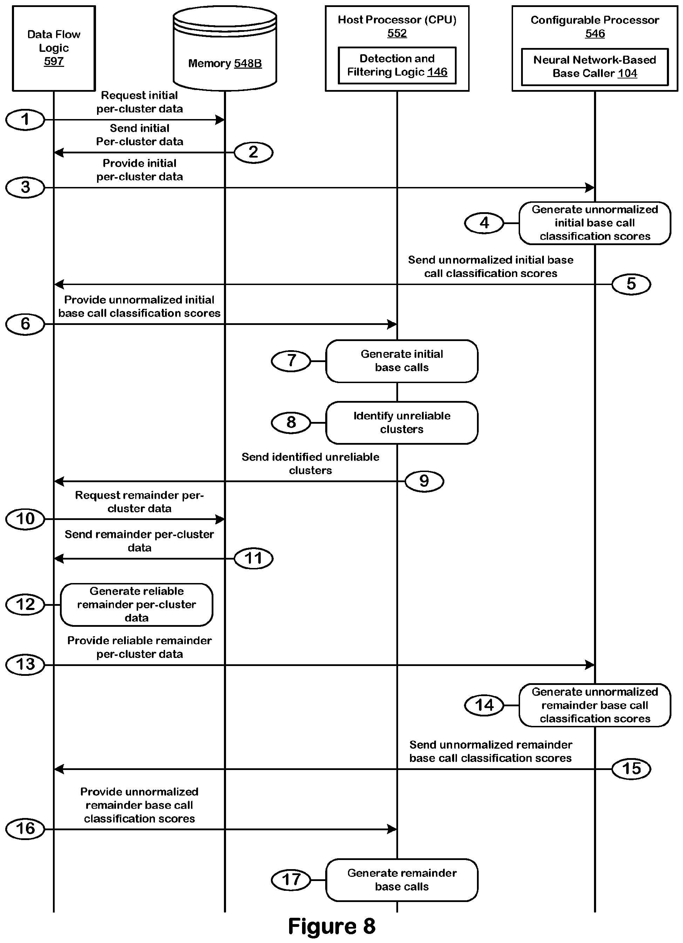

Figure 8 shows yet another implementation of the disclosed data flow logic that enables the host processor to filter unreliable clusters based on base calls predicted by the neural network running on the configurable processor, and further uses data identifying the unreliable clusters to generate reliable remainder per-cluster data.

At action 1, the data flow logic 597 requests initial per-cluster data from the memory 548B. Per-cluster data refers to image patches that are extracted from sequencing images and centered around a target cluster to be base called. A center pixel of the images patches contains a center of the target cluster. The images patches, in addition to the target cluster, also depict signal from additional clusters adjacent to the target cluster. Initial per-cluster data includes image patches that are centered at the target clusters and depict intensity emissions of the target clusters at initial sequencing cycles of a sequencing run, i.e., a first subset of sequencing cycles of the sequencing run, as discussed above. For example, the initial per-cluster data can include image patches for the first 25 sequencing cycles (initial sequencing cycles) of the sequencing run.

At action 2, the memory 548B sends the initial per-cluster data to the data flow logic 597.

At action 3, the data flow logic 597 provides the initial per-cluster data to the configurable processor 546.

At action 4, the neural network-based base caller 104, running on the configurable processor 546, generates initial intermediate representations (e.g., feature maps) from the initial per-cluster data (e.g., by processing the initial per-cluster data through its spatial and temporal convolution layers), and produces initial base call classification scores for the plurality of clusters and for the initial sequencing cycles based on the initial intermediate representations. In one implementation, the initial base call classification scores are unnormalized, for example, they are not subjected to exponential normalization by a softmax function.

At action 5, the configurable processor 546 sends the unnormalized initial base call classification scores to the data flow logic 597.

At action 6, the data flow logic 597 provides the unnormalized initial base call classification scores to the host processor 552.

At action 7, the host processor 552 normalizes the unnormalized initial base call classification scores (e.g., by applying the softmax function), and generates normalized initial base call classification scores, i.e., initial base calls.

At action 8, the detection and filtering logic 146, running on the host processor 552, uses the normalized initial base call classification scores/initial base calls to identify unreliable clusters in the plurality of clusters based on generating filter values, as discussed above in the section titled "Detecting and Filtering Unreliable Clusters".

At action 9, the host processor 552 sends data identifying the unreliable clusters to the data flow logic 597. The unreliable clusters can be identified by instrument ID, the run number on the instrument, the flow cell ID, the lane number, the tile number, the X coordinate of the cluster, the Y coordinate of the cluster, and unique molecular identifiers (UMIs).

At action 10, the data flow logic 597 requests remainder per-cluster data from the memory 548B. Remainder per-cluster data includes image patches that are centered at the target clusters and depict intensity emissions of the target clusters at remainder sequencing cycles of the sequencing run, i.e., those sequencing cycles of the sequencing run that do not include the first subset of sequencing cycles of the sequencing run, as discussed above. For example, the remainder per-cluster data can include image patches for the 26 to 100 sequencing cycles (the last 75 sequencing cycles) of a 100-cycle sequencing run.

At action 11, the memory 548B sends the remainder per-cluster data to the data flow logic 597.

At action 12, the data flow logic 597 uses the data identifying the unreliable clusters to generate reliable remainder per-cluster data by removing, from the remainder per-cluster data, per-cluster data that represents the unreliable clusters.

At action 13, the data flow logic 597 provides the reliable remainder per-cluster data to the configurable processor 546.

At action 14, the neural network-based base caller 104, running on the configurable processor 546, produces remainder base call classification scores only for those clusters in the plurality of clusters that are not the unreliable clusters and for the remainder sequencing cycles, thereby bypasses production of the remainder base call classification scores for the unreliable clusters. In one implementation, the remainder base call classification scores are unnormalized, for example, they are not subjected to exponential normalization by a softmax function.

At action 15, the configurable processor 546 sends the unnormalized remainder base call classification scores to the data flow logic 597.

At action 16, the data flow logic 597 provides the unnormalized remainder base call classification scores to the host processor 552.

At action 17, the host processor 552 normalizes the unnormalized remainder base call classification scores (e.g., by applying the softmax function), and generates normalized remainder base call classification scores, i.e., remainder base.

### Technical Improvement

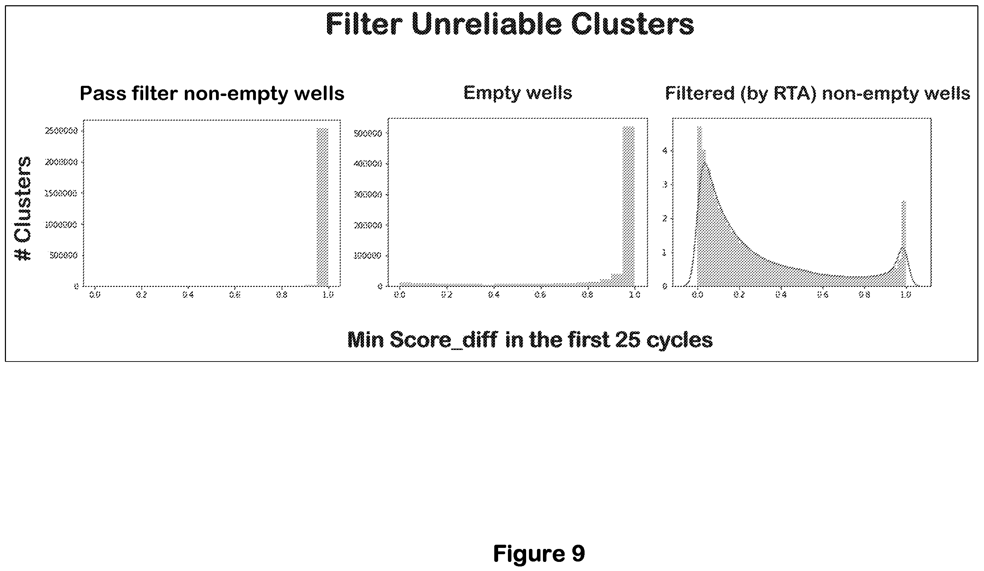

Figures 9, 10, 11, 12, and 13 show results of comparative analysis of detection of empty and non-empty wells by the data flow logic disclosed herein and referred to as "DeepRTA" versus Illumina's traditional base caller called Real-Time Analysis (RTA) software.

In Figure 9, in all three plots, x-axis is the minimum of score difference across the first 25 cycles, where the score difference is the result of subtracting the second highest likelihood from the highest likelihood. The y-axis is the number of clusters in one tile. The first plot is the result on clusters which passed RTA chastity filter. The middle plot is for empty wells (no clusters in these nanowells according to RTA). The third plot is the result on clusters which failed RTA chastity filter. Majority of clusters detected as unreliable using RTA chastity filter have at least one instance of low score_diff in the first 25 cycles.

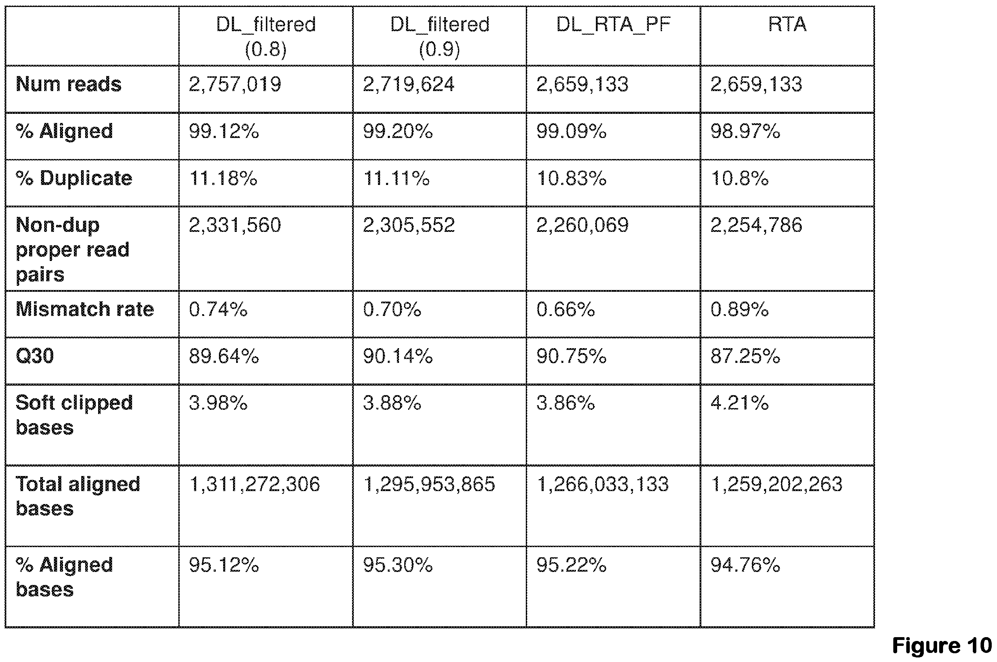

In Figure 10, alignment metrics of one tile are depicted. The last column shows the alignment metrics using reliable clusters based on RTA chastity filter and RTA base calls. The next to last one shows the alignment metrics using reliable clusters based on RTA chastity filter and DeepRTA base calls. The first two columns are alignment metrics using DeepRTA base calls and reliable clusters based on the disclosed data flow logic, where the threshold is 0.8 (first column), or 0.9 (second column), and 2 out of the first 25 cycles should have not met the threshold to be considered unreliable.

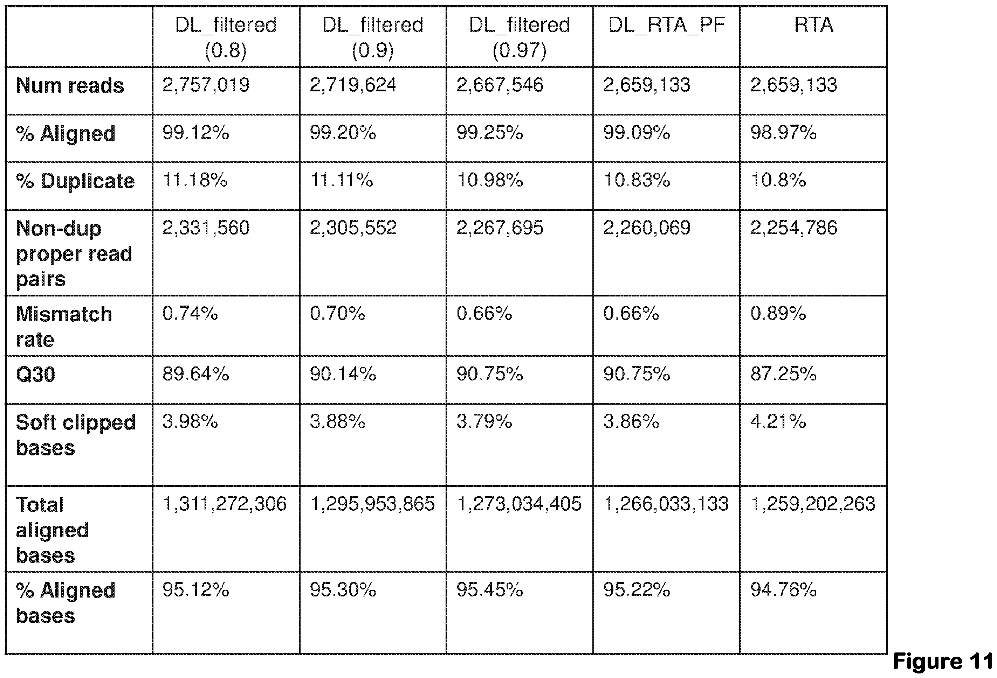

In Figure 11, similar to Figure 10, a 0.97 threshold is added. Using the disclosed data flow logic and threshold 0.97, more clusters are detected as reliable compared to using RTA chastity filter, while maintaining similar (or better) alignment metrics.

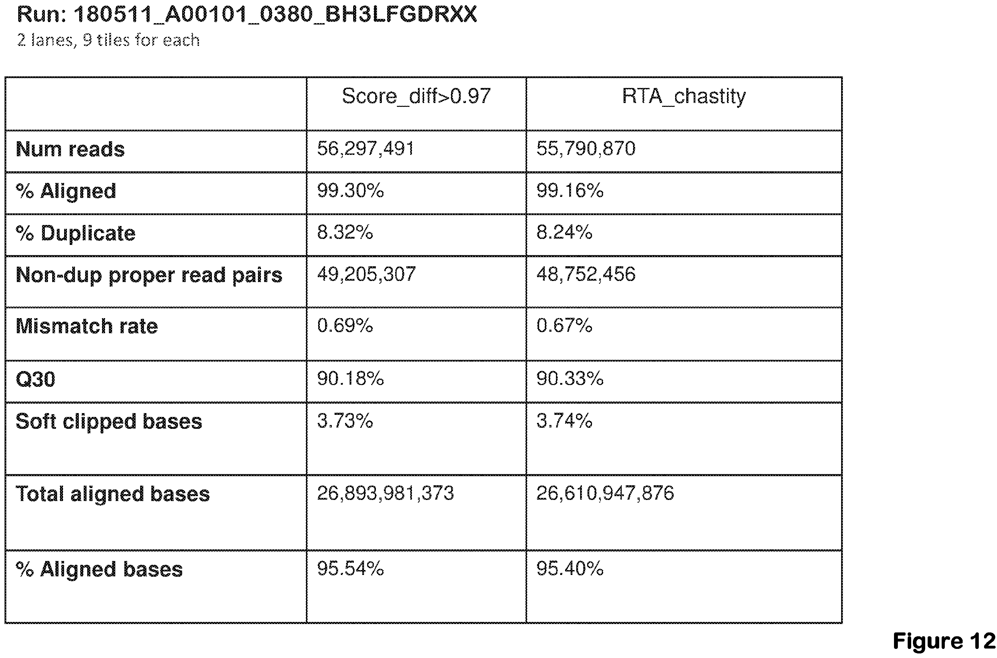

Figure 12 shows alignment metrics based on data from 18 tiles of a sequencing run. First column is DeepRTA base calls and reliable clusters using threshold of 0.97 (subtracting second highest from the highest likelihood), and 2 out of the first 25 cycles should be below the threshold to be considered unreliable. The last column is DeepRTA base calls and reliable clusters using RTA chastity filter. Using the disclosed data flow logic, more clusters are detected as reliable compared to using RTA chastity filter, while maintaining similar alignment metrics.

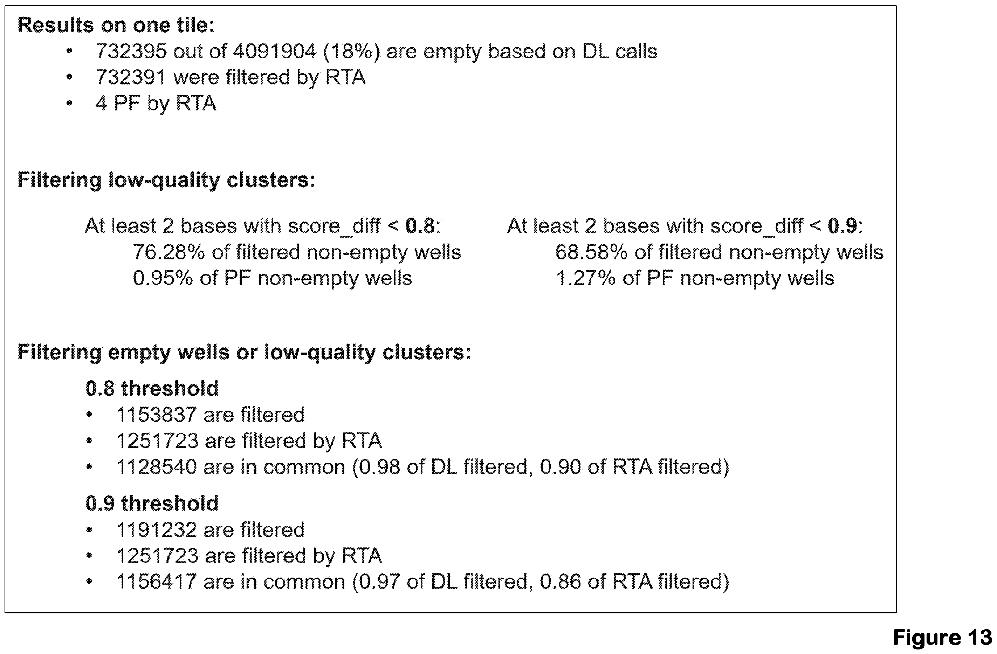

Figure 13 illustrates comparison of RTA chastity filter and the disclosed data flow logic using different thresholds. A large percentage of unreliable clusters detected by the disclosed data flow logic were also detected as unreliable by RTA chastity filter.

### Computer System

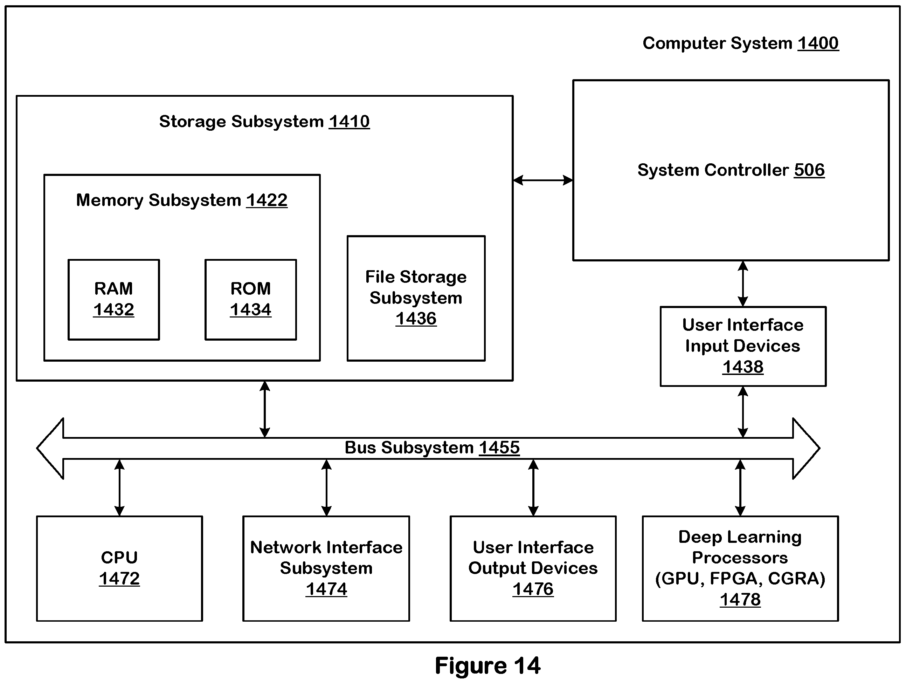

Figure 14 is a computer system 1400 that can be used by the sequencing system 500A to implement the base calling techniques disclosed herein. Computer system 1400 includes at least one central processing unit (CPU) 1472 that communicates with a number of peripheral devices via bus subsystem 1455. These peripheral devices can include a storage subsystem 858 including, for example, memory devices and a file storage subsystem 1436, user interface input devices 1438, user interface output devices 1476, and a network interface subsystem 1474. The input and output devices allow user interaction with computer system 1400. Network interface subsystem 1474 provides an interface to outside networks, including an interface to corresponding interface devices in other computer systems.

In one implementation, the system controller 506 is communicably linked to the storage subsystem 1410 and the user interface input devices 1438.

User interface input devices 1438 can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system 1400.

User interface output devices 1476 can include a display subsystem, a printer, a fax machine, or non-visual displays such as audio output devices. The display subsystem can include an LED display, a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system 1400 to the user or to another machine or computer system.

Storage subsystem 858 stores programming and data constructs that provide the functionality of some or all of the modules and methods described herein. These software modules are generally executed by deep learning processors 1478.

Deep learning processors 1478 can be graphics processing units (GPUs), field-programmable gate arrays (FPGAs), application-specific integrated circuits (ASICs), and/or coarse-grained reconfigurable architectures (CGRAs). Deep learning processors 1478 can be hosted by a deep learning cloud platform such as Google Cloud Platform™, Xilinx™, and Cirrascale™. Examples of deep learning processors 1478 include Google's Tensor Processing Unit (TPU)™, rackmount solutions like GX4 Rackmount Series™, GX14 Rackmount Series™, NVIDIA DGX-1™, Microsoft' Stratix V FPGA™, Graphcore's Intelligent Processor Unit (IPU)™, Qualcomm's Zeroth Platform™ with Snapdragon processors™, NVIDIA's Volta™, NVIDIA's DRIVE PX™, NVIDIA's JETSON TX1/TX2 MODULE™, Intel's Nirvana™, Movidius VPU™, Fujitsu DPI™, ARM's DynamicIQ™, IBM TrueNorth™, Lambda GPU Server with Testa V100s™, and others.

Memory subsystem 1422 used in the storage subsystem 858 can include a number of memories including a main random access memory (RAM) 1432 for storage of instructions and data during program execution and a read only memory (ROM) 1434 in which fixed instructions are stored. A file storage subsystem 1436 can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem 1436 in the storage subsystem 858, or in other machines accessible by the processor.

Bus subsystem 1455 provides a mechanism for letting the various components and subsystems of computer system 1400 communicate with each other as intended. Although bus subsystem 1455 is shown schematically as a single bus, alternative implementations of the bus subsystem can use multiple busses.

Computer system 1400 itself can be of varying types including a personal computer, a portable computer, a workstation, a computer terminal, a network computer, a television, a mainframe, a server farm, a widely-distributed set of loosely networked computers, or any other data processing system or user device. Due to the ever changing nature of computers and networks, the description of computer system 1400 depicted in Figure 14 is intended only as a specific example for purposes of illustrating the preferred implementations of the present invention. Many other configurations of computer system 1400 are possible having more or less components than the computer system depicted in Figure 14.

**Particular Implementations**

We describe various implementations of filtering clusters based on artificial intelligence-predicted base calls. One or more features of an implementation can be combined with the base implementation, and can be practiced as a system, method, or article of manufacture. Implementations that are not mutually exclusive are taught to be combinable. One or more features of an implementation can be combined with other implementations. This disclosure periodically reminds the user of these options. Omission from some implementations of recitations that repeat these options should not be taken as limiting the combinations taught in the preceding sections - these recitations are hereby incorporated forward by reference into each of the following implementations.

In one implementation, the technology disclosed proposes a computer-implemented method of identifying unreliable clusters to improve accuracy and efficiency of neural network-based base calling. The technology disclosed accesses per-cycle cluster data for a plurality of clusters and for a first subset of sequencing cycles of a sequencing run.

The technology disclosed uses a neural network-based base caller to base call each cluster in the plurality of clusters at each sequencing cycle in the first subset of sequencing cycles. This includes processing the per-cycle cluster data through the neural network-based base caller and generating intermediate representations of the per-cycle cluster data. This further includes processing the intermediate representations though an output layer and producing a per-cluster, per-cycle probability quadruple for each cluster and for each sequencing cycle. A particular per-cluster, per-cycle probability quadruple identifies probabilities of a base incorporated in a particular cluster at a particular sequencing cycle being A, C, T, and G.

The technology disclosed determines a filter value for each per-cluster, per-cycle probability quadruple based on the probabilities it identifies, thereby generating a sequence of filter values for each cluster.

The technology disclosed identifies those clusters in the plurality of clusters as unreliable clusters whose sequences of filter values contain "N" number of filter values below a threshold "M".

The technology disclosed bypasses base calling the unreliable clusters at a remainder of sequencing cycles of the sequencing run, thereby using the neural network-based base caller to base call, at the remainder of sequencing cycles, only those clusters in the plurality of clusters that are not identified as the unreliable clusters.

**Claims**

1. A computer-implemented method of identifying unreliable clusters to improve accuracy and efficiency of base calling, the computer-implemented method including:

   accessing per-cycle cluster data for a plurality of clusters and for a first subset of sequencing cycles of a sequencing run;
   base calling of a multi-pixel image through a neural network-based base caller each cluster in the plurality of clusters at each sequencing cycle in the first subset of sequencing cycles, including:

   processing the per-cycle cluster data and generating intermediate representations of the per-cycle cluster data through the neural network-based base caller, and
   processing the intermediate representations through a normalization function of an output layer of the neural network-based base caller and producing a per-cluster, per-cycle probability quadruple for each cluster and for each sequencing cycle, wherein a particular per-cluster, per-cycle probability quadruple identifies probabilities of a base incorporated in a particular cluster at a particular sequencing cycle being A, C, T, and G;

   using the identified probabilities to determine a filter value as a function of a highest probability that determines a base call for each per-cluster, per-cycle probability quadruple, thereby generating a sequence of filter values for each cluster;
   identifying as unreliable clusters those clusters in the plurality of clusters whose sequences of filter values contain at least "N" number of filter values below a threshold "M"; and
   bypassing base calling of the unreliable clusters at a remainder of sequencing cycles of the sequencing run, thereby base calling, at the remainder of sequencing cycles, only those clusters in the plurality of clusters that are not identified as the unreliable clusters.

2. The computer-implemented method of claim 1, wherein the filter value for a per-cluster, per-cycle probability quadruple is determined based on an arithmetic operation involving one or more of the probabilities.

3. The computer-implemented method of claim 2, wherein the arithmetic operation is subtraction.

4. The computer-implemented method of claims 1-3, wherein the filter value for the per-cluster, per-cycle probability quadruple is determined by subtracting a second highest one of the probabilities from a highest one of the probabilities.

5. The computer-implemented method of claim 2, wherein the arithmetic operation is division.

6. The computer-implemented method of claims 4-5, wherein the filter value for the per-cluster, per-cycle probability quadruple is determined as a ratio of the highest one of the probabilities to the second highest one of the probabilities.

7. The computer-implemented method of claim 2, wherein the arithmetic operation is addition.

8. The computer-implemented method of claim 2, wherein the arithmetic operation is multiplication.

9. The computer-implemented method of claims 1-8, wherein the at least "N" number of filter values ranges from 1 to 5.

10. The computer-implemented method of claims 1-9, wherein the threshold "M" ranges from 0.5 to 0.99.

11. The computer-implemented method of claims 1-10, wherein the first subset of sequencing cycles includes 1 to 25 sequencing cycles of the sequencing run.

12. The computer-implemented method of claims 1-11, wherein the first subset of sequencing cycles includes 1 to 50 sequencing cycles of the sequencing run.

13. The computer-implemented method of claims 1-12, wherein the output layer is a softmax layer and the probabilities in the per-cluster, per-cycle probability quadruple are exponentially normalized classification scores that sum to unity.

14. The computer-implemented method of claims 1-13, wherein the unreliable clusters are indicative of empty, polyclonal, and dim wells on a patterned flow cell.

15. A system including one or more processors coupled to memory, the memory loaded with computer instructions that, when executed on the one or more processors, cause the system to perform a method according to any one of claims 1-14.


**Patentansprüche**

1. Computerimplementiertes Verfahren zum Identifizieren unzuverlässiger Cluster, um die Genauigkeit und Effizienz des Base-Calling zu verbessern, wobei das computerimplementierte Verfahren umfasst:

Zugreifen auf Clusterdaten pro Zyklus für eine Vielzahl von Clustern und für eine erste Teilmenge von Sequenzierungszyklen eines Sequenzierungslaufs;
Base-Calling eines Mehrpixelbildes durch einen auf einem neuronalen Netz basierenden Base-Caller für jeden Cluster in der Vielzahl von Clustern bei jedem Sequenzierungszyklus in der ersten Teilmenge von Sequenzierungszyklen, einschließlich:

Verarbeiten der Clusterdaten pro Zyklus und Erzeugen von Zwischendarstellungen der Clusterdaten pro Zyklus durch den auf neuronalen Netzwerken basierenden Base-Caller, und
Verarbeiten der Zwischendarstellungen durch eine
Normalisierungsfunktion einer Ausgabeschicht des auf einem neuronalen Netz basierenden Base-Callers und Erzeugen eines Vierfachen der Wahrscheinlichkeiten pro Cluster und pro Zyklus für jeden Cluster und für jeden Sequenzierungszyklus, wobei ein bestimmtes Vierfache der Wahrscheinlichkeiten pro Cluster und pro Zyklus Wahrscheinlichkeiten einer Base identifiziert, die in einem bestimmten Cluster bei einem bestimmten Sequenzierungszyklus enthalten ist und A, C, T und G ist;
Verwenden der identifizierten Wahrscheinlichkeiten zum Bestimmen eines Filterwerts als Funktion einer höchsten Wahrscheinlichkeit, die einen Base-Call für jedes Vierfache der Wahrscheinlichkeiten pro Cluster und pro Zyklus bestimmt, wodurch eine Sequenz von Filterwerten für jeden Cluster erzeugt wird;
Identifizieren jener Cluster in der Vielzahl von Clustern als unzuverlässige Cluster, deren Sequenzen von Filterwerten mindestens "N" Anzahl von Filterwerten unterhalb eines Schwellenwertes "M" enthalten; und
Umgehen des Base-Calling der unzuverlässigen Cluster bei einem Rest von Sequenzierungszyklen des Sequenzierungslaufs, wodurch bei dem Rest von Sequenzierungszyklen nur diejenigen Cluster in der Vielzahl von Clustern Base-Calling erhalten, die nicht als die unzuverlässigen Cluster identifiziert werden.

2. Computerimplementiertes Verfahren nach Anspruch 1, wobei der Filterwert für ein Vierfaches der Wahrscheinlichkeit pro Cluster und pro Zyklus auf der Grundlage einer arithmetischen Operation bestimmt wird, die eine oder mehrere der Wahrscheinlichkeiten beinhaltet.

3. Computerimplementiertes Verfahren nach Anspruch 2, wobei die arithmetische Operation eine Subtraktion ist.

4. Computerimplementiertes Verfahren nach Anspruch 1 bis 3, wobei der Filterwert für das Vierfache der Wahrscheinlichkeiten pro Cluster und pro Zyklus durch Subtraktion einer zweithöchsten der Wahrscheinlichkeiten von einer höchsten der Wahrscheinlichkeiten bestimmt wird.

5. Computerimplementiertes Verfahren nach Anspruch 2, wobei die arithmetische Operation eine Division ist.

6. Computerimplementiertes Verfahren nach Anspruch 4 bis 5, wobei der Filterwert für das Vierfache der Wahrscheinlichkeiten pro Cluster und pro Zyklus als Verhältnis der höchsten der Wahrscheinlichkeiten zu der zweithöchsten der Wahrscheinlichkeiten bestimmt wird.

7. Computerimplementiertes Verfahren nach Anspruch 2, wobei die arithmetische Operation Addition ist.

8. Computerimplementiertes Verfahren nach Anspruch 2, bei dem die arithmetische Operation Multiplikation ist.

9. Computerimplementiertes Verfahren nach einem der Ansprüche 1 bis 8, wobei die mindestens "N" Anzahl von Filterwerten im Bereich von 1 bis 5 liegt.

10. Computerimplementiertes Verfahren nach einem der Ansprüche 1-9, wobei der Schwellenwert "M" im Bereich von 0,5 bis 0,99 liegt.

11. Computerimplementiertes Verfahren nach Anspruch 1-10, wobei die erste Teilmenge von Sequenzierungszyklen 1 bis 25 Sequenzierungszyklen des Sequenzierungslaufs umfasst.

12. Computerimplementiertes Verfahren nach Anspruch 1-11, wobei die erste Teilmenge von Sequenzierungszyklen 1 bis 50 Sequenzierungszyklen des Sequenzierungslaufs umfasst.

13. Computerimplementiertes Verfahren nach Anspruch 1-12, wobei die Ausgabeschicht eine Softmax-Schicht ist und die Wahrscheinlichkeiten in dem Vierfachen der Wahrscheinlichkeiten pro Cluster und pro Zyklus exponentiell normalisierte Klassifizierungsergebnisse sind, die sich zu Eins summieren.

14. Computerimplementiertes Verfahren nach Anspruch 1-13, wobei die unzuverlässigen Cluster auf leere, polyklonale und undeutliche Vertiefungen auf einer gemusterten Durchflusszelle hinweisen.

15. System einschließlich eines oder mehrerer Prozessoren, die mit einem Speicher gekoppelt sind, wobei der Speicher mit Computeranweisungen geladen ist, die, wenn sie auf dem einen oder den mehreren Prozessoren ausgeführt werden, das System veranlassen, ein Verfahren nach einem der Ansprüche 1-14 durchzuführen.

## Revendications

1. Procédé mis en oeuvre par ordinateur d'identification de grappes non fiables afin d'améliorer la précision et l'efficacité d'appel de base, le procédé mis en oeuvre par ordinateur comportant :

l'accès aux données de grappes par cycle pour une pluralité de grappes et pour un premier sous-ensemble de cycles de séquençage d'une série de séquençages;
l'appel de base d'une image multipixel par le biais d'un appelant de base basé sur un réseau neuronal pour chaque grappe de la pluralité de grappes à chaque cycle de séquençage dans le premier sous-ensemble de cycles de séquençage, comportant :

le traitement des données de grappes par cycle et la génération de représentations intermédiaires des données de grappes par cycle par le biais de l'appelant de base basé sur un réseau neuronal, et
le traitement des représentations intermédiaires par le biais d'une fonction de normalisation d'une couche de sortie de l'appelant de base basé sur un réseau neuronal et la production d'un quadruplet de probabilité par grappe et par cycle pour chaque grappe et pour chaque cycle de séquençage, dans lequel un quadruplet de probabilité particulier par grappe et par cycle identifie des probabilités qu'une base incorporée dans une grappe particulière à un cycle de séquençage particulier comme étant A, C, T et G ;
l'utilisation des probabilités identifiées pour déterminer une valeur de filtre en fonction d'une probabilité la plus élevée qui détermine un appel de base pour chaque quadruplet de probabilité par grappe et par cycle, générant ainsi une séquence de valeurs de filtre pour chaque grappe ;
l'identification comme grappes non fiables des grappes de la pluralité de grappes dont les séquences de valeurs de filtre contiennent au moins « N » nombre de valeurs de filtre en dessous d'un seuil « M » ; et
le contournement de l'appel de base des grappes non fiables au niveau d'un reste de cycles de séquençage de la série de séquençage, en appelant ainsi de base, lors du reste de cycles de séquençage, uniquement les grappes de la pluralité de grappes qui ne sont pas identifiées comme les grappes non fiables.

**2.** Procédé mis en oeuvre par ordinateur selon la revendication 1, dans lequel la valeur de filtre pour un quadruplet de probabilité par grappe et par cycle est déterminée sur la base d'une opération arithmétique impliquant une ou plusieurs des probabilités.

**3.** Procédé mis en oeuvre par ordinateur selon la revendication 2, dans lequel l'opération arithmétique est une soustraction.

**4.** Procédé mis en oeuvre par ordinateur selon les revendications 1 à 3, dans lequel la valeur de filtre pour le quadruplet de probabilité par grappe et par cycle est déterminée en soustrayant la seconde probabilité la plus élevée de la première probabilité la plus élevée.

**5.** Procédé mis en oeuvre par ordinateur selon la revendication 2, dans lequel l'opération arithmétique est une division.

**6.** Procédé mis en oeuvre par ordinateur selon les revendications 4 à 5, dans lequel la valeur de filtre pour le quadruplet de probabilité par grappe et par cycle est déterminée comme un rapport entre la probabilité la plus élevée et la seconde probabilité la plus élevée.

**7.** Procédé mis en oeuvre par ordinateur selon la revendication 2, dans lequel l'opération arithmétique est une addition.

**8.** Procédé mis en oeuvre par ordinateur selon la revendication 2, dans lequel l'opération arithmétique est une multiplication.

**9.** Procédé mis en oeuvre par ordinateur selon les revendications 1 à 8, dans lequel le nombre « N » au moins de valeurs de filtre est compris entre 1 et 5.

**10.** Procédé mis en oeuvre par ordinateur selon les revendications 1 à 9, dans lequel le seuil « M » est compris entre 0,5 et 0,99.

**11.** Procédé mis en oeuvre par ordinateur selon les revendications 1 à 10, dans lequel le premier sous-ensemble de cycles de séquençage comporte de 1 à 25 cycles de séquençage de la série de séquençage.

**12.** Procédé mis en oeuvre par ordinateur selon les revendications 1 à 11, dans lequel le premier sous-ensemble de cycles de séquençage comprend de 1 à 50 cycles de séquençage de la série de séquençage.

**13.** Procédé mis en oeuvre par ordinateur selon les revendications 1 à 12, dans lequel la couche de sortie est une couche softmax et les probabilités dans le quadruplet de probabilité par grappe et par cycle sont des scores de classification normalisés de manière exponentielle dont la somme est égale à l'unité.

**14.** Procédé mis en oeuvre par ordinateur selon les revendications 1 à 13, dans lequel les grappes non fiables indiquent des puits vides, polyclonaux et peu lumineux sur une cuve de circulation à motifs.

**15.** Système comportant un ou plusieurs processeurs couplés à une mémoire, la mémoire étant chargée d'instructions informatiques qui, lorsqu'elles sont exécutées sur le ou les processeurs, amènent le système à réaliser le procédé selon l'une quelconque des revendications 1 à 14.

**Figure 1**

# Softmax Function

**Figure 2A**

EP 4 205 123 B1

Color Key:

| |
|---|
| Brown |
| Magenta |
| Blue |
| Green |
| Purple |

**202** — Cluster 1

| **212** Sequencing Cycles | 1 | 2 | 3 | ... | $s$ |
|---|---|---|---|---|---|
| A | 0.80 | 0.20 | 0.05 | ... | 0.13 |
| C | 0.14 | 0.70 | 0.60 | ... | 0.25 |
| T | 0.05 | 0.08 | 0.25 | ... | 0.40 |
| G | 0.01 | 0.02 | 0.10 | ... | 0.22 |
| **232** Filter Values (score difference) | 0.66 | 0.50 | 0.35 | ... | 0.15 |

**222** Probability Quadruples

**Figure 2B**

EP 4 205 123 B1

Threshold "M" = 0.5

Number of Filter Values "N" => 2

**302**

| Cluster 1 Filter Values | 0.25 | 0.55 | 0.53 | 0.65 | 0.18 | 0.71 | 0.77 | 0.76 | 0.82 | 0.94 | 0.56 | 0.89 |

→ Stop Base Calling

Unreliable Cluster

**312**

| Cluster 2 Filter Values | 0.99 | 0.52 | 0.51 | 0.59 | 0.77 | 0.86 | 0.79 | 0.40 | 0.49 | 0.69 | 0.14 | 0.60 |

→ Stop Base Calling

Unreliable Cluster

**322**

| Cluster 3 Filter Values | 0.60 | 0.59 | 0.51 | 0.77 | 0.80 | 0.91 | 0.78 | 0.89 | 0.40 | 0.79 | 0.56 | 0.69 |

→ Continue Base Calling

Reliable Cluster

Color Key:

| Pink |
| Purple |
| Green |

**Figure 3**

| | |
|---|---|
| 402 | accessing per-cycle cluster data for a plurality of clusters and for a first subset of sequencing cycles of a sequencing run |
| 412 | base calling each cluster in the plurality of clusters at each sequencing cycle in the first subset of sequencing cycles |
| 422 | processing the per-cycle cluster data and generating intermediate representations of the per-cycle cluster data |
| 432 | processing the intermediate representations though an output layer and producing a per-cluster, per-cycle probability quadruple for each cluster and for each sequencing cycle |
| 442 | determining a filter value for each per-cluster, per-cycle probability quadruple based on the probabilities it identifies |
| 452 | identifying those clusters in the plurality of clusters as unreliable clusters whose sequences of filter values contain "N" number of filter values below a threshold "M" |
| 462 | bypassing base calling the unreliable clusters at a remainder of sequencing cycles of the sequencing run |

## Figure 4

**Figure 5A**

Figure 5B

Figure 5C

**Figure 6**

**Figure 7**

**Figure 8**

Figure 9

| | DL_filtered (0.8) | DL_filtered (0.9) | DL_RTA_PF | RTA |
|---|---|---|---|---|
| **Num reads** | 2,757,019 | 2,719,624 | 2,659,133 | 2,659,133 |
| **% Aligned** | 99.12% | 99.20% | 99.09% | 98.97% |
| **% Duplicate** | 11.18% | 11.11% | 10.83% | 10.8% |
| **Non-dup proper read pairs** | 2,331,560 | 2,305,552 | 2,260,069 | 2,254,786 |
| **Mismatch rate** | 0.74% | 0.70% | 0.66% | 0.89% |
| **Q30** | 89.64% | 90.14% | 90.75% | 87.25% |
| **Soft clipped bases** | 3.98% | 3.88% | 3.86% | 4.21% |
| **Total aligned bases** | 1,311,272,306 | 1,295,953,865 | 1,266,033,133 | 1,259,202,263 |
| **% Aligned bases** | 95.12% | 95.30% | 95.22% | 94.76% |

**Figure 10**

|  | DL_filtered (0.8) | DL_filtered (0.9) | DL_filtered (0.97) | DL_RTA_PF | RTA |
|---|---|---|---|---|---|
| Num reads | 2,757,019 | 2,719,624 | 2,667,546 | 2,659,133 | 2,659,133 |
| % Aligned | 99.12% | 99.20% | 99.25% | 99.09% | 98.97% |
| % Duplicate | 11.18% | 11.11% | 10.98% | 10.83% | 10.8% |
| Non-dup proper read pairs | 2,331,560 | 2,305,552 | 2,267,695 | 2,260,069 | 2,254,786 |
| Mismatch rate | 0.74% | 0.70% | 0.66% | 0.66% | 0.89% |
| Q30 | 89.64% | 90.14% | 90.75% | 90.75% | 87.25% |
| Soft clipped bases | 3.98% | 3.88% | 3.79% | 3.86% | 4.21% |
| Total aligned bases | 1,311,272,306 | 1,295,953,865 | 1,273,034,405 | 1,266,033,133 | 1,259,202,263 |
| % Aligned bases | 95.12% | 95.30% | 95.45% | 95.22% | 94.76% |

**Figure 11**

## Run: 180511_A00101_0380_BH3LFGDRXX
2 lanes, 9 tiles for each

| | Score_diff>0.97 | RTA_chastity |
|---|---|---|
| **Num reads** | 56,297,491 | 55,790,870 |
| **% Aligned** | 99.30% | 99.16% |
| **% Duplicate** | 8.32% | 8.24% |
| **Non-dup proper read pairs** | 49,205,307 | 48,752,456 |
| **Mismatch rate** | 0.69% | 0.67% |
| **Q30** | 90.18% | 90.33% |
| **Soft clipped bases** | 3.73% | 3.74% |
| **Total aligned bases** | 26,893,981,373 | 26,610,947,876 |
| **% Aligned bases** | 95.54% | 95.40% |

**Figure 12**

**Results on one tile:**
- 732395 out of 4091904 (18%) are empty based on DL calls
- 732391 were filtered by RTA
- 4 PF by RTA

**Filtering low-quality clusters:**

At least 2 bases with score_diff < **0.8**:
    76.28% of filtered non-empty wells
    0.95% of PF non-empty wells

At least 2 bases with score_diff < **0.9**:
    68.58% of filtered non-empty wells
    1.27% of PF non-empty wells

**Filtering empty wells or low-quality clusters:**

**0.8 threshold**
- 1153837 are filtered
- 1251723 are filtered by RTA
- 1128540 are in common (0.98 of DL filtered, 0.90 of RTA filtered)

**0.9 threshold**
- 1191232 are filtered
- 1251723 are filtered by RTA
- 1156417 are in common (0.97 of DL filtered, 0.86 of RTA filtered)

**Figure 13**

**Figure 14**

EP 4 205 123 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62821602 A
- US 62821618 A
- US 62821681 A
- US 62821724 A
- US 62821766 A
- NL 2023310
- NL 2023311
- NL 2023312
- NL 2023314
- NL 2023316
- US 62849091 A
- US 62849132 A
- US 62849133 A
- US 62979384 A
- US 62979414 A
- US 62979385 A
- US 62979412 A
- US 62979411 A
- US 62979399 A
- US 2018274023 A1
- US 20120020537 A1
- WO 04015497 A
- US 7057026 B
- WO 9106675 A
- WO 07123744 A
- US 7329492 B
- US 7211414 B
- US 7315019 B
- US 7405251 B
- US 2005014705052 A
- US 61535294 A
- US 61619575 B
- US 62420012 A
- US 20070166705 A1
- US 200601563901 A1
- US 20060240439 A1
- US 200602514714709 A1
- WO 05065514 A
- US 2005014700900 A1
- WO 0605B199 A
- WO 0701470251 A
- US 7541444 B
- US 741414716 B
- US 7427673 B
- US 7566537 B
- US 7592435 B
- WO 0714535365 A

**Non-patent literature cited in the description**

- naiveBayesCall: An Efficient Model-Based Base-Calling Algorithm for High-Throughput Sequencing. **WEI-CHUN KAO et al.** Research In Computational Molecular Biology. Springer, 25 April 2010, 233-247
- **BENTLEY et al.** *Nature*, 2005, vol. 456, 53-59